# EUROPEAN PATENT APPLICATION

(11) **EP 4 293 680 A1**
(43) Date of publication of application: **20.12.2023**
(21) Application number: 23178939.7
(22) Date of filing: 13.06.2023
(51) Int. Cl.: G16H 40/20, G16H 40/40

(54) **SYSTEMS AND METHODS FOR MONITORING SURGICAL WORKFLOW AND PROGRESS**

(30) Priority: 14.06.2022 US 202263366398 P
(71) Applicant: Stryker Corporation, Kalamazoo, MI 49002 (US)
(72) Inventor: TIWARY, Vijay Kumar, Kalamazoo, 49002 (US); HASTINGS, Sean Victor, Kalamazoo, 49002 (US); BHARDWAJ, Gaurav, Kalamazoo, 49002 (US); SHARMA, Chitrank, Kalamazoo, 49002 (US); GUREVICH, Lina, Kalamazoo, 49002 (US); COUGHLAN, Heather Marlene, Kalamazoo, 49002 (US)
(74) Representative: V.O.

(57) **Abstract**

The present disclosure relates generally to improving operating room scheduling and resource management efficiency, and more specifically to techniques for monitoring various aspects of an operating room. An exemplary method for adjusting a surgical schedule specifying timing information for one or more surgeries to occur in an operating room comprises: receiving one or more images of the operating room captured by one or more cameras; detecting one or more surgical milestones of a plurality of predefined surgical milestones using one or more trained machine-learning models based on the received one or more images, wherein the one or more machine learning models are trained using a plurality of training images depicting the one or more surgical milestones; and adjusting, based on the detected one or more surgical milestones, the surgical schedule specifying timing information for the one or more surgeries to occur in the operating room.

## Description

### CROSS-REFERENCE TO RELATED APPLICATIONS

This application claims the benefit of U.S. Provisional Application No. 63/366,398, filed June 14, 2022, the entire contents of which are hereby incorporated by reference herein.

### FIELD

The present disclosure relates generally to improving surgery safety, and more specifically to techniques for monitoring various aspects of an operating room.

### BACKGROUND

Many surgical departments do not utilize modern technology for scheduling of operating room (OR) suites and departments, procedures, and resources. It is not uncommon for departmental grease boards or whiteboards to be utilized in this setting for scheduling and management of various OR activities. This methodology can be inefficient and inaccurate with respect to predicting schedule changes and medical resource planning. Further, it is dependent on active manual management of the surgical schedule and medical resources, diverting staff attention away from patient care. It further detracts from efficient preparation of pre-operation patients, room turnover (e.g., sanitation), and communication with patients and their families. As such, there is a need for improved systems and methods that provide for more efficient and effective management of medical resources and surgical scheduling, thus improving surgical safety and patient care.

### SUMMARY

Disclosed herein are exemplary devices, apparatuses, systems, methods, and non-transitory storage media for monitoring various aspects of an operating room and adjusting a surgical schedule. An exemplary system can adjust a surgical schedule specifying timing information for one or more surgeries to occur in an operating room by: receiving one or more images of the operating room captured by one or more cameras; detecting one or more surgical milestones of a plurality of predefined surgical milestones using one or more trained machine-learning models based on the received one or more images; and adjusting, based on the detected one or more surgical milestones, the surgical schedule specifying timing information for the one or more surgeries to occur in the operating room. The machine learning model can be trained using a plurality of training images depicting the one or more surgical milestones. The one or more images can be captured by cameras in the operating room. The one or more images can include images captured by one or more surgical devices (e.g., endoscopes). By utilizing images captured by cameras generally installed in the operating room in conjunction with information from surgical devices, the system may provide more accurate and realistic identification of surgical milestones and estimation of time between surgical milestones (e.g., surgery started, surgery closing). For example, a cholecystectomy may last anywhere from one to three hours (or even longer), depending on the patient, the anatomy, and the surgeon's skills. Therefore, being able to correctly identify all the surgical milestones specific to this procedure (using intra-operative endoscopic images among other types of images) may result in a more accurate overall estimation of the remaining time.

The system can be configured to display the adjusted surgical schedule. The system can display the adjusted schedule on a monitor in the operating room. The system can display the adjusted schedule on a display in a monitoring area (e.g., at a central control center for monitoring multiple operating rooms). The system can display the adjusted schedule as a message (e.g., a text message, a notification) on a mobile device.

The system can be configured to identify one or more medical resources in use during a surgery (e.g., based on the detected surgical milestones). The system can e.g. determine, based on the one or more medical resources in use during the surgery, the availability of the identified one or more medical resources for a scheduled surgical procedure. The one or more medical resources can include medical equipment, surgical staff, or any combination thereof. The system may generate an alert based on the determined availability of the identified one or more medical resources. Adjusting the surgical schedule can include adjusting the surgical schedule based on the determined availability of the identified one or more medical resources. The system can be configured to adjust the surgical schedule for one or more operating rooms (e.g., a plurality of operation rooms) based on the determined availability of the identified one or more medical resources. The system can be configured to allocate medical resources, such as equipment and/or surgical staff, to one or more operating rooms based on detected surgical milestones in the one or more operating rooms.

The system can be configured to determine whether a detected surgical milestone of the detected one or more surgical milestones is a charting event or an alerting event. In accordance with a determination that the detected surgical milestone is a charting event, the system records the detected surgical milestone in a database, such as an electronic medical record. In accordance with a determination that the detected surgical milestone is an alerting event, the system generates an alert (e.g., alerting surgical staff to conduct a timeout).

Detecting the one or more surgical milestones can include obtaining, from the one or more trained machine-learning models, one or more detected objects or events; and determining, based upon the one or more detected objects or events, the one or more surgical milestones. The system may automatically update, based on one or more detected events, a surgical timeout checklist specifying a list of surgical action items. During the time-out, the operating room team may review the patient's identity, the procedure, the surgical site, and various other information before proceeding with the procedure. The surgical timeout checklist may specify surgical action items related to surgical preparation activities.

The system can determine, based upon the detected one or more surgical milestones, whether to schedule an emergency surgery in the operating room. In accordance with a determination that an emergency surgery should be scheduled, the system schedules the surgery. In accordance with a determination that an emergency surgery should not be scheduled, the system foregoes scheduling the emergency surgery. The system can be configured to allocate medical resources, such as equipment and/or surgical staff, to an operating room for the emergency surgery. The system can be configured to adjust the surgical schedule for the remaining operating room(s) based on the availability of the medical resources and/or the detected one or more surgical milestones.

The system may determine, based on the detected one or more surgical milestones, whether the operating room is available for a next surgery. In accordance with a determination that an operating room is available for surgery, the system generates an alert indicating that the room is available for the next surgery. In accordance with a determination that an operating room is not available for surgery, the system foregoes generation of the alert indicating that the room is available for the next surgery. The system may be configured to generate an information message to surgical staff, a patient and/or family of patient containing information regarding a scheduled start time of a surgery and/or preparation for a surgery. The system can be configured to generate an updated information message to surgical staff, a patient and/or family of patient containing updated information regarding a scheduled start time of a surgery and/or preparation for a surgery, in case of a change in the surgical schedule.

Accordingly, examples of the present disclosure ensure efficient scheduling of surgical procedures and efficient management of medical resources in an operating room by monitoring various aspects of the operating room. As such, the system can relieve the burden placed on surgical staff to perform duties such as tracking various surgical events and/or milestones so that greater attention can be given to the care of the patient, thus improving patient care and surgical safety.

An exemplary method for adjusting a surgical schedule specifying timing information for one or more surgeries to occur in an operating room comprises: receiving one or more images of the operating room captured by one or more cameras; detecting one or more surgical milestones of a plurality of predefined surgical milestones using one or more trained machine-learning models based on the received one or more images, wherein the one or more machine learning models are trained using a plurality of training images depicting the one or more surgical milestones; and adjusting, based on the detected one or more surgical milestones, the surgical schedule specifying timing information for the one or more surgeries to occur in the operating room.

The method can further comprise displaying the adjusted surgical schedule.

Detecting the one or more surgical milestones can comprise: obtaining, from the one or more trained machine-learning models, one or more detected objects or events; and determining, based upon the one or more detected objects or events, the one or more surgical milestones.

Adjusting the surgical schedule can includes: obtaining an original schedule, wherein the original schedule is based on historical time information of the plurality of predefined surgical milestones; comparing the original schedule with time information of the one or more detected surgical milestones; and updating, based on the comparison, the surgical schedule.

The method can further comprise determining, based upon the detected one or more surgical milestones, whether preparation of pre-operation patients should be accelerated or delayed; and in accordance with a determination that preparation of pre-operation patients should be accelerated, accelerating preparation of pre-operation patients; in accordance with a determination that preparation of pre-operation patients should be delayed, delaying preparation of pre-operation patients.

The method can further comprise identifying, based upon the one or more images, one or more medical resources in use during a surgery; and determining, based on the identified one or more medical resources in use during the surgery, the availability of the identified one or more medical resources for a scheduled surgical procedure.

The one or more medical resources can comprise medical equipment, surgical staff, or any combination thereof.

The method can further comprises generating an alert based on the determined availability of the identified one or more medical resources.

Adjusting the surgical schedule can comprise adjusting the surgical schedule based on the determined availability of the identified one or more medical resources. Adjusting the surgical schedule can comprise adjusting the surgical schedule for one or more operating rooms based on the determined availability of the identified one or more medical resources. Adjusting the surgical schedule can comprise rescheduling a surgery from one operating room to another based on detected surgical milestones in the one or more operating rooms. The method can comprise allocating medical resources, such as equipment and/or surgical staff, to one or more operating rooms based on detected surgical milestones in the one or more operating rooms.

The method can further comprise determining whether a detected surgical milestone of the detected one or more surgical milestones is a charting event or an alerting event; in accordance with a determination that the detected surgical milestone is a charting event, recording the detected surgical milestone in a database; and in accordance with a determination that the detected surgical milestone is an alerting event, generating an alert.

The database can be an electronic medical record.

The method can further comprise automatically updating, based on the one or more detected objects or events, a surgical timeout checklist specifying a list of surgical action items.

The method can further comprise determining, based upon the detected one or more surgical milestones, whether to schedule an emergency surgery in the operating room; in accordance with a determination that an emergency surgery should be scheduled, scheduling the surgery; in accordance with a determination that an emergency surgery should not be scheduled, foregoing scheduling the emergency surgery. The method can comprise allocating medical resources, such as equipment and/or surgical staff, to an operating room for the emergency surgery. The method can comprise adjusting the surgical schedule for the remaining operating room(s) based on the availability of the medical resources and/or the detected one or more surgical milestones.

The method can further comprise determining, based on the detected one or more surgical milestones, whether the operating room is available for a next surgery; in accordance with a determination that an operating room is available for surgery, generating an alert indicating that the room is available for the next surgery; in accordance with a determination that an operating room is not available for surgery, foregoing generation of the alert indicating that the room is available for the next surgery. The method can comprise generating an information message to surgical staff, a patient and/or family of patient containing information regarding a scheduled start time of a surgery and/or preparation for a surgery. The method can comprise generating an updated information message to surgical staff, a patient and/or family of patient containing updated information regarding a scheduled start time of a surgery and/or preparation for a surgery, in case of a change in the surgical schedule.

The one or more objects can include: a stretcher, a patient, a surgeon, an anesthesiologist, the surgeon's hand, a surgical assistant, a scrub nurse, a technician, a nurse, a scalpel, sutures, a staple gun, a door to a sterile room, a door to a non-sterile corridor, a retractor, a clamp, an endoscope, an electrocautery tool, an intubation mask, a surgical mask, a C-Arm, an Endoscopic Equipment Stack, an anesthesia machine, an anesthesia cart, a fluid management system, a waste management system, a waste disposal receptacle, an operating table, surgical table accessories, an equipment boom, an anesthesia boom, an endoscopic equipment cart, surgical lights, a case cart, a sterile back table, a sterile mayo stand, a cleaning cart, an X-Ray device, an imaging device, a trocar, a surgical drape, operating room floor, EKG leads, ECG leads, bed linens, a blanket, a heating blanket, a lap belt, safety straps, a pulse oximeter, a blood pressure machine, an oxygen mask, an IV, or any combination thereof.

The one or more events can include: whether the surgical lights are turned off, whether the operating table is vacant, whether the bed linens are wrinkled, whether the bed linens are stained, whether the operating table is wiped down, whether a new linen is applied to the operating table, whether a first sterile case cart is brought into the operating room, whether a new patient chart is created, whether instrument packs are distributed throughout the operating room, whether booms and suspended equipment are repositioned, whether the operating table is repositioned, whether a nurse physically exposes instrumentation by unfolding linen or paper, or opening instrumentation containers using a sterile technique, whether the scrub nurse entered the operating room, whether the technician entered the operating room, whether the scrub nurse is donning a gown, whether the circulating nurse is securing the scrub nurse's gown, whether the scrub nurse is donning gloves using the sterile technique, whether the sterile back table or the sterile mayo stand is being set with sterile instruments, whether the patient is wheeled into the operating room on a stretcher, whether the patient is wheeled into the operating room on a wheel chair, whether the patient walked into the operating room, whether the patient is carried into the operating room, whether the patient is transferred to the operating table, whether the patient is covered with the blanket, whether the lap belt is applied to the patient, whether the pulse oximeter is placed on the patient, whether the EKG leads are applied to the patient, whether the ECG leads are applied to the patient, whether the blood pressure cuff is applied to the patient, whether a surgical sponge and instrument count is conducted, whether a nurse announces a timeout, whether a surgeon announces a timeout, whether an anesthesiologist announces a timeout, whether activities are stopped for a timeout, whether the anesthesiologist gives the patient the oxygen mask, whether the patient is sitting and leaning over with the patient's back cleaned and draped, whether the anesthesiologist inspects the patient's anatomy with a long needle, whether the anesthesiologist injects medication into the patient's back, whether the anesthesiologist indicates that the patient is ready for surgery, whether the patient is positioned for a specific surgery, whether required surgical accessories are placed on a table, whether padding is applied to the patient, whether the heating blanket is applied to the patient, whether the safety straps are applied to the patient, whether a surgical site on the patient is exposed, whether the surgical lights are turned on, whether the surgical lights are positioned to illuminate the surgical site, whether the scrub nurse is gowning the surgeon, whether the scrub nurse is gloving the surgeon, whether skin antiseptic is applied, whether the surgical site is draped, whether sterile handles are applied to the surgical lights, whether a sterile team member is handing off tubing to a non-sterile team member, whether a sterile team member is handing off electrocautery to a non-sterile team member, whether the scalpel is handed to the surgeon, whether a sponge is handed to the surgeon, whether an incision is made, whether the sutures are handed to the surgeon, whether the staple gun is handed to the surgeon, whether the scrub nurse is handing a sponge to a sponge collection basin, whether an incision is closed, whether dressing is applied to cover a closed incision, whether the surgical lights are turned off, whether the anesthesiologist is waking the patient, whether the patient is returned to a supine position, whether extubation is occurring, whether instruments are being placed on the case cart, whether a garbage bag is being tied up, whether the bed linens are collected and tied up, whether the operating table surface is cleaned, whether the operating room floor is being mopped, whether the patient is being transferred to a stretcher, whether the patient is being brought out of the operating room, whether the surgical table is dressed with a clean linen, whether a second sterile case cart is brought into the operating room, or any combination thereof.

The plurality of predefined milestones can include: whether an operating room is ready, whether operating room setup has started, whether the scrub nurse or the technician are donning gloves, whether operating room equipment is being set up, whether the patient is brought in to the operating room, whether the patient is ready for intubation or anesthesia, whether a timeout is occurring, whether the timeout has occurred, whether the patient is intubated or anesthetized, whether the patient has been prepped and draped for surgery, whether the patient is ready for surgery, whether a surgery site prep is complete, whether a surgery has started, whether the surgery is closing, whether a dressing is applied to the patient, whether the surgery is stopped, whether the patient is brought out of the operating room, whether the operating room is being cleaned, whether the operating room is clean, or any combination thereof.

Determining whether the operating room is ready can be based on whether the operating room table is empty, whether the surgical lights are turned off, whether the bed linens are wrinkled, whether the operating table is wiped down, whether the operating room floor is mopped, or whether a new linen is applied to the operating table.

Determining whether the patient is prepped and draped for surgery can be based on: whether the patient is positioned for a specific surgery, whether required surgical accessories are placed on a table, whether padding is applied to the patient, whether the heating blanket is applied to the patient, whether the safety straps are applied to the patient, or whether a surgical site on the patient is exposed.

A trained machine-learning model of the one or more trained machine-learning models can be an object detection algorithm, a video action detection algorithm, a surgical milestone detection algorithm, an anomaly detection algorithm, or any combination thereof.

A trained machine-learning model of the one or more trained machine-learning models can be a neural network model.

The one or more trained machine-learning models can be trained using a plurality of annotated images.

A trained machine-learning model of the one or more trained machine-learning models can be a deep learning model trained using annotated surgical video information, wherein the annotated surgical video information includes annotations of at least one of the plurality of predefined surgical milestones.

The method can further comprise training, based on the detected one or more surgical milestones, a machine-learning model configured to predict timing information of a future surgery.

The timing information can comprise a predicted duration of the future surgery, a predicted duration of a surgical milestone of the future surgery, or any combination thereof.

The method can further comprise identifying one or more people associated with a surgery in the operating room; and obtaining timing information associated with the identified one or more people.

The surgical schedule can be adjusted based on a weighted combination of the timing information associated with the identified one or more people and the detected one or more surgical milestones.

Identifying the one or more people can comprise: retrieving data associated with the surgery from a database.

Identifying the one or more people can comprise: detecting the one or more people using a trained machine-learning model based on the received one or more images.

The plurality of predefined surgical milestones can comprise one or more preoperative milestones, one or more intraoperative milestones, and one or more postoperative milestones.

An exemplary system for adjusting a surgical schedule specifying timing information for one or more surgeries to occur in an operating room comprises: one or more processors; a memory; and one or more programs, wherein the one or more programs are stored in the memory and configured to be executed by the one or more processors, the one or more programs including instructions for: receiving one or more images of the operating room captured by one or more cameras; detecting one or more surgical milestones of a plurality of predefined surgical milestones using one or more trained machine-learning models based on the received one or more images, wherein the one or more machine learning models are trained using a plurality of training images depicting the one or more surgical milestones; and adjusting, based on the detected one or more surgical milestones, the surgical schedule specifying timing information for the one or more surgeries to occur in the operating room.

The one or more programs can further include instructions for: displaying the adjusted surgical schedule.

Detecting the one or more surgical milestones can comprise: obtaining, from the one or more trained machine-learning models, one or more detected objects or events; and determining, based upon the one or more detected objects or events, the one or more surgical milestones.

Adjusting the surgical schedule can include: obtaining an original schedule, wherein the original schedule is based on historical time information of the plurality of predefined surgical milestones; comparing the original schedule with time information of the one or more detected surgical milestones; and updating, based on the comparison, the surgical schedule.

The one or more programs can further include instructions for: determining, based upon the detected one or more surgical milestones, whether preparation of pre-operation patients should be accelerated or delayed; and in accordance with a determination that preparation of pre-operation patients should be accelerated, accelerating preparation of pre-operation patients; in accordance with a determination that preparation of pre-operation patients should be delayed, delaying preparation of pre-operation patients.

The one or more programs can further include instructions for: identifying, based upon the one or more images, one or more medical resources in use during a surgery; and determining, based on the identified one or more medical resources in use during the surgery, the availability of the identified one or more medical resources for a scheduled surgical procedure.

The one or more medical resources can comprise medical equipment, surgical staff, or any combination thereof.

The one or more programs can further include instructions for: generating an alert based on the determined availability of the identified one or more medical resources.

Adjusting the surgical schedule can comprise adjusting the surgical schedule based on the determined availability of the identified one or more medical resources.

The one or more programs can further include instructions for: determining whether a detected surgical milestone of the detected one or more surgical milestones is a charting event or an alerting event; in accordance with a determination that the detected surgical milestone is a charting event, recording the detected surgical milestone in a database; and in accordance with a determination that the detected surgical milestone is an alerting event, generating an alert.

The database can be an electronic medical record.

The one or more programs can further include instructions for: automatically updating, based on the one or more detected objects or events, a surgical timeout checklist specifying a list of surgical action items.

The one or more programs can further include instructions for: determining, based upon the detected one or more surgical milestones, whether to schedule an emergency surgery in the operating room; in accordance with a determination that an emergency surgery should be scheduled, scheduling the surgery; in accordance with a determination that an emergency surgery should not be scheduled, foregoing scheduling the emergency surgery.

The one or more programs can further include instructions for: determining, based on the detected one or more surgical milestones, whether the operating room is available for a next surgery; in accordance with a determination that an operating room is available for surgery, generating an alert indicating that the room is available for the next surgery; in accordance with a determination that an operating room is not available for surgery, foregoing generation of the alert indicating that the room is available for the next surgery.

The one or more objects can include: a stretcher, a patient, a surgeon, an anesthesiologist, the surgeon's hand, a surgical assistant, a scrub nurse, a technician, a nurse, a scalpel, sutures, a staple gun, a door to a sterile room, a door to a non-sterile corridor, a retractor, a clamp, an endoscope, an electrocautery tool, an intubation mask, a surgical mask, a C-Arm, an Endoscopic Equipment Stack, an anesthesia machine, an anesthesia cart, a fluid management system, a waste management system, a waste disposal receptacle, an operating table, surgical table accessories, an equipment boom, an anesthesia boom, an endoscopic equipment cart, surgical lights, a case cart, a sterile back table, a sterile mayo stand, a cleaning cart, an X-Ray device, an imaging device, a trocar, a surgical drape, operating room floor, EKG leads, ECG leads, bed linens, a blanket, a heating blanket, a lap belt, safety straps, a pulse oximeter, a blood pressure machine, an oxygen mask, an IV, or any combination thereof.

The one or more events can include: whether the surgical lights are turned off, whether the operating table is vacant, whether the bed linens are wrinkled, whether the bed linens are stained, whether the operating table is wiped down, whether a new linen is applied to the operating table, whether a first sterile case cart is brought into the operating room, whether a new patient chart is created, whether instrument packs are distributed throughout the operating room, whether booms and suspended equipment are repositioned, whether the operating table is repositioned, whether a nurse physically exposes instrumentation by unfolding linen or paper, or opening instrumentation containers using a sterile technique, whether the scrub nurse entered the operating room, whether the technician entered the operating room, whether the scrub nurse is donning a gown, whether the circulating nurse is securing the scrub nurse's gown, whether the scrub nurse is donning gloves using the sterile technique, whether the sterile back table or the sterile mayo stand is being set with sterile instruments, whether the patient is wheeled into the operating room on a stretcher, whether the patient is wheeled into the operating room on a wheel chair, whether the patient walked into the operating room, whether the patient is carried into the operating room, whether the patient is transferred to the operating table, whether the patient is covered with the blanket, whether the lap belt is applied to the patient, whether the pulse oximeter is placed on the patient, whether the EKG leads are applied to the patient, whether the ECG leads are applied to the patient, whether the blood pressure cuff is applied to the patient, whether a surgical sponge and instrument count is conducted, whether a nurse announces a timeout, whether a surgeon announces a timeout, whether an anesthesiologist announces a timeout, whether activities are stopped for a timeout, whether the anesthesiologist gives the patient the oxygen mask, whether the patient is sitting and leaning over with the patient's back cleaned and draped, whether the anesthesiologist inspects the patient's anatomy with a long needle, whether the anesthesiologist injects medication into the patient's back, whether the anesthesiologist indicates that the patient is ready for surgery, whether the patient is positioned for a specific surgery, whether required surgical accessories are placed on a table, whether padding is applied to the patient, whether the heating blanket is applied to the patient, whether the safety straps are applied to the patient, whether a surgical site on the patient is exposed, whether the surgical lights are turned on, whether the surgical lights are positioned to illuminate the surgical site, whether the scrub nurse is gowning the surgeon, whether the scrub nurse is gloving the surgeon, whether skin antiseptic is applied, whether the surgical site is draped, whether sterile handles are applied to the surgical lights, whether a sterile team member is handing off tubing to a non-sterile team member, whether a sterile team member is handing off electrocautery to a non-sterile team member, whether the scalpel is handed to the surgeon, whether a sponge is handed to the surgeon, whether an incision is made, whether the sutures are handed to the surgeon, whether the staple gun is handed to the surgeon, whether the scrub nurse is handing a sponge to a sponge collection basin, whether an incision is closed, whether dressing is applied to cover a closed incision, whether the surgical lights are turned off, whether the anesthesiologist is waking the patient, whether the patient is returned to a supine position, whether extubation is occurring, whether instruments are being placed on the case cart, whether a garbage bag is being tied up, whether the bed linens are collected and tied up, whether the operating table surface is cleaned, whether the operating room floor is being mopped, whether the patient is being transferred to a stretcher, whether the patient is being brought out of the operating room, whether the surgical table is dressed with a clean linen, whether a second sterile case cart is brought into the operating room, or any combination thereof.

The plurality of predefined milestones can include: whether an operating room is ready, whether operating room setup has started, whether the scrub nurse or the technician are donning gloves, whether operating room equipment is being set up, whether the patient is brought in to the operating room, whether the patient is ready for intubation or anesthesia, whether a timeout is occurring, whether the timeout has occurred, whether the patient is intubated or anesthetized, whether the patient has been prepped and draped for surgery, whether the patient is ready for surgery, whether a surgery site prep is complete, whether a surgery has started, whether the surgery is closing, whether a dressing is applied to the patient, whether the surgery is stopped, whether the patient is brought out of the operating room, whether the operating room is being cleaned, whether the operating room is clean, or any combination thereof.

Determining whether the operating room is ready can be based on whether the operating room table is empty, whether the surgical lights are turned off, whether the bed linens are wrinkled, whether the operating table is wiped down, whether the operating room floor is mopped, or whether a new linen is applied to the operating table.

Determining whether the patient is prepped and draped for surgery can be based on: whether the patient is positioned for a specific surgery, whether required surgical accessories are placed on a table, whether padding is applied to the patient, whether the heating blanket is applied to the patient, whether the safety straps are applied to the patient, or whether a surgical site on the patient is exposed.

A trained machine-learning model of the one or more trained machine-learning models can be an object detection algorithm, a video action detection algorithm, a surgical milestone detection algorithm, an anomaly detection algorithm, or any combination thereof.

A trained machine-learning model of the one or more trained machine-learning models can be a neural network model.

The one or more trained machine-learning models can be trained using a plurality of annotated images.

A trained machine-learning model of the one or more trained machine-learning models can be a deep learning model trained using annotated surgical video information, wherein the annotated surgical video information includes annotations of at least one of the plurality of predefined surgical milestones.

The one or more programs further can include instructions for: training, based on the detected one or more surgical milestones, a machine-learning model configured to predict timing information of a future surgery.

The timing information can comprise a predicted duration of the future surgery, a predicted duration of a surgical milestone of the future surgery, or any combination thereof.

The one or more programs can further include instructions for: identifying one or more people associated with a surgery in the operating room; and obtaining timing information associated with the identified one or more people.

The surgical schedule can be adjusted based on a weighted combination of the timing information associated with the identified one or more people and the detected one or more surgical milestones.

Identifying the one or more people can comprise: retrieving data associated with the surgery from a database.

Identifying the one or more people can comprise: detecting the one or more people using a trained machine-learning model based on the received one or more images.

The plurality of predefined surgical milestones can comprise one or more preoperative milestones, one or more intraoperative milestones, and one or more postoperative milestones.

An exemplary non-transitory computer-readable storage medium stores one or more programs, the one or more programs comprising instructions, which when executed by one or more processors of an electronic device, cause the electronic device to perform any of the methods described herein.

### BRIEF DESCRIPTION OF THE FIGURES

FIG. 1 illustrates an exemplary view of a medical care area.
FIG. 2 illustrates an exemplary process for adjusting a surgical schedule.
FIG. 3A illustrates an exemplary machine-learning model used to detect surgical milestones.
FIG. 3B illustrates an exemplary machine-learning model used to detect objects and/or events, which are in turn used to detect surgical milestones.
FIG. 4 illustrates an exemplary series of surgical milestones.
FIG. 5 depicts an exemplary electronic device.

### DETAILED DESCRIPTION

Disclosed herein are exemplary devices, apparatuses, systems, methods, and non-transitory storage media for monitoring various aspects of an operating room and adjusting a surgical schedule. An exemplary system can adjust a surgical schedule specifying timing information for one or more surgeries to occur in an operating room by: receiving one or more images of the operating room captured by one or more cameras; detecting one or more surgical milestones of a plurality of predefined surgical milestones using one or more trained machine-learning models based on the received one or more images; and adjusting, based on the detected one or more surgical milestones, the surgical schedule specifying timing information for the one or more surgeries to occur in the operating room. The machine learning model can be trained using a plurality of training images depicting the one or more surgical milestones. The system can be configured to adjust a surgical schedule specifying timing information for one or more surgeries to occur in one or more operating rooms by receiving one or more images of the operating rooms captured by one or more cameras. A milestone may refer to a phase or period of time during a surgical workflow (e.g., surgical phase), or a specific time point during the surgical workflow. A surgical milestone can refer to a preoperative activity, an intraoperative activity, or a postoperative activity, as discussed herein.

The system can be configured to obtain the original surgical schedule from one or more databases, such as an electronic medical record (EMR) system, an electronic health record (EHR) system, and/or a health information system (HIS). The system can display the adjusted surgical schedule. The system can display the adjusted schedule on a monitor in the operating room. Alternatively, or additionally, the system displays the adjusted schedule on a display in a monitoring area (e.g., at a central control center for monitoring multiple operating rooms). The system can display the adjusted schedule as a message (e.g., a text message, a notification) on a mobile device. Instead of automatically adjusting the schedule, the system can allows the medical staff in charge to accept or override the schedule adjustment. For example, the system may display a proposed adjustment to the schedule and prompt a user to accept or reject the proposed adjustment.

The system can identify one or more medical resources in use during a surgery (e.g., based on the detected surgical milestones). The system can e.g. determine, based on the one or more medical resources in use during the surgery, the availability of the identified one or more medical resources for a scheduled surgical procedure. The one or more medical resources can include medical equipment, surgical staff, or any combination thereof. The system may generate an alert based on the determined availability of the identified one or more medical resources. Adjusting the surgical schedule can include adjusting the surgical schedule based on the determined availability of the identified one or more medical resources. The system can be configured to adjust the surgical schedule for one or more operating rooms based on the determined availability of the identified one or more medical resources. The system can be configured to allocate medical resources, such as equipment and/or surgical staff, to one or more operating rooms based on detected surgical milestones in the one or more operating rooms.

The system can determine whether a detected surgical milestone of the detected one or more surgical milestones is a charting event or an alerting event. In accordance with a determination that the detected surgical milestone is a charting event, the system records the detected surgical milestone in a database, such as an electronic medical record. In accordance with a determination that the detected surgical milestone is an alerting event, the system generates an alert (e.g., alerting surgical staff to conduct a timeout).

Detecting the one or more surgical milestones can include obtaining, from the one or more trained machine-learning models, one or more detected objects or events; and determining, based upon the one or more detected objects or events, the one or more surgical milestones. The system can e.g. automatically update, based on one or more detected events, a surgical timeout checklist specifying a list of surgical action items.

The system can determine, based upon the detected one or more surgical milestones, whether to schedule an emergency surgery in the operating room. In accordance with a determination that an emergency surgery should be scheduled, the system schedules the surgery. In accordance with a determination that an emergency surgery should not be scheduled, the system foregoes scheduling the emergency surgery. The system can be configured to allocate medical resources, such as equipment and/or surgical staff, to an operating room for the emergency surgery. The system can be configured to adjust the surgical schedule for the remaining operating room(s) based on the availability of the medical resources and/or the detected one or more surgical milestones. The system may issue an alert regarding the scheduled emergency surgery. The alert may be sent to the medical staff members assigned to prepare for the surgery, procure resources for the surgery, perform the surgery, etc. As discussed herein, the alert may be displayed on a display in the operating room and/or in a central control room, and may be transmitted to the medical staff via text messages.

The system can determine, based on the detected one or more surgical milestones, whether the operating room is available for a next surgery. In accordance with a determination that an operating room is available for surgery, the system generates an alert indicating that the room is available for the next surgery. In accordance with a determination that an operating room is not available for surgery, the system foregoes generation of the alert indicating that the room is available for the next surgery. In some examples, the next surgery may be performed for a different patient. In some examples, the next surgery may be performed for the same patient. For example, a mastectomy may need to be performed immediately after a breast reconstruction procedure. In this setup, the surgeons performing the two procedures may be two different people and be assisted by two different surgical crews. By automatically monitoring the progress of the mastectomy, the completion time for the surgery may be estimated and the plastic surgeon and crew may be alerted accordingly. The system may be configured to generate an information message to surgical staff, a patient and/or family of patient containing information regarding a scheduled start time of a surgery and/or preparation for a surgery. The system can be configured to generate an updated information message to surgical staff, a patient and/or family of patient containing updated information regarding a scheduled start time of a surgery and/or preparation for a surgery, in case of a change in the surgical schedule.

Accordingly, examples of the present disclosure ensure efficient scheduling of surgical procedures and efficient management of medical resources in an operating room, or a plurality of operating rooms, by monitoring various aspects of the operating room. As such, the system can relieve the burden placed on surgical staff to perform duties such as tracking various surgical events and/or milestones so that greater attention can be given to the care of the patient, thus improving patient care and surgical safety. Further, prompt alerts and updates by the system may serve to alleviate family concerns caused by unanticipated delays or other adjustments to the surgical schedule, in contrast to conventional systems that may be incapable of detecting surgical milestones, adjusting surgical schedules, and/or providing such alerts.

The following description sets forth exemplary methods, parameters, and the like. It should be recognized, however, that such description is not intended as a limitation on the scope of the present disclosure but is instead provided as a description of exemplary examples.

Although the following description uses terms "first," "second," etc. to describe various elements, these elements should not be limited by the terms. These terms are only used to distinguish one element from another. For example, a first graphical representation could be termed a second graphical representation, and, similarly, a second graphical representation could be termed a first graphical representation, without departing from the scope of the various described examples. The first graphical representation and the second graphical representation are both graphical representations, but they are not the same graphical representation.

The terminology used in the description of the various described examples herein is for the purpose of describing particular examples only and is not intended to be limiting. As used in the description of the various described examples and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes," "including," "comprises," and/or "comprising," when used in this specification, specify the presence of stated features, integers, steps, operations, elements, and/or components, but do not preclude the presence or addition of one or more other features, integers, steps, operations, elements, components, and/or groups thereof.

The term "if' is, optionally, construed to mean "when" or "upon" or "in response to determining" or "in response to detecting," depending on the context. Similarly, the phrase "if it is determined" or "if [a stated condition or event] is detected" is, optionally, construed to mean "upon determining" or "in response to determining" or "upon detecting [the stated condition or event]" or "in response to detecting [the stated condition or event]," depending on the context.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating" or the like, refer to the action and processes of a computer system, or similar electronic computing device, that manipulates and transforms data represented as physical (electronic) quantities within the computer system memories or registers or other such information storage, transmission, or display devices.

The present disclosure in some examples also relates to a device for performing the operations herein. This device may be specially constructed for the required purposes, or it may comprise a general purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

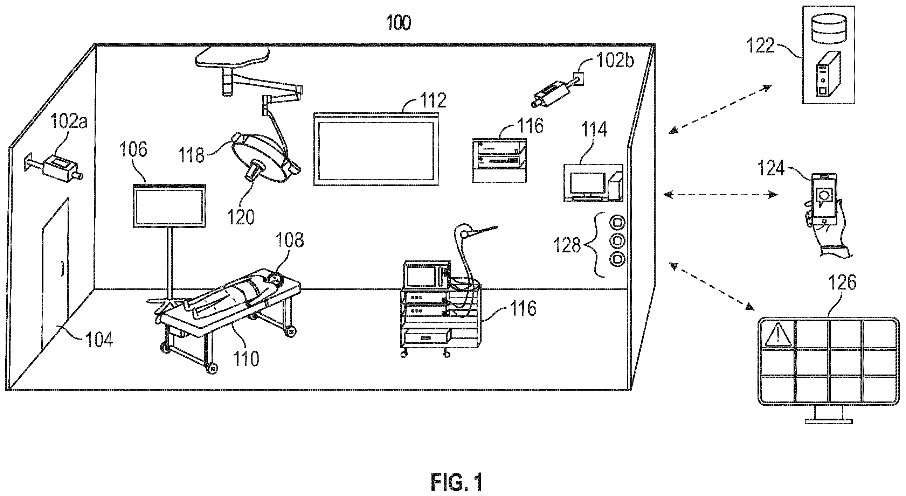

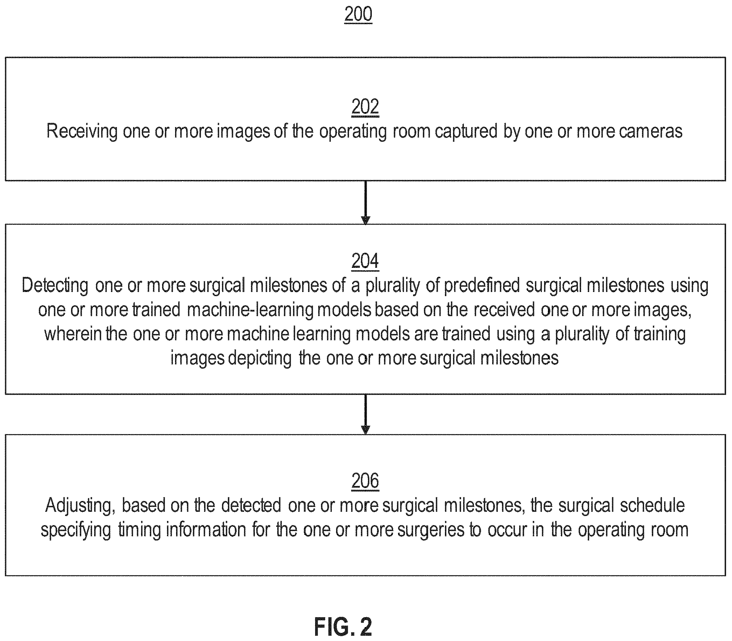

FIG. 1 illustrates an exemplary view of a medical care area 100. In the illustrated example, the medical care area 100 is an operating room where surgical operations are carried out in an antiseptic environment. The medical care area 100 includes one or more doors such as door 104, one or more medical charts, such as chart 106, one or more case carts, a patient, such as patient 108, an operating table, such as operating room table 110, one or more operating room monitors, such as operating room monitor 112, one or more computer systems, such as computer system 114, one or more pieces of medical equipment, such as medical equipment 116, one or more surgical lights, such as surgical light 118, one or more cameras, such as cameras 102a, 102b, and 120, and one or more sensors, such as sensors 128 (e.g., for monitoring various environmental factors. Inside or outside operating room 100, there may be one or more electronic medical record systems (EMR systems), such as electronic medical record system 122, one or more mobile devices, such as mobile device 124, and one or more displays, such as display 126. It should be understood that the aforementioned list is exemplary and there may be different, additional or fewer items in or associated with the operating room. For instance, in some examples, the medical care area may include multiple doors, for example, a door that connects to a sterile room where sterile equipment and staff enter/exit through and another door that connects to a non-sterile corridor where patient enters/exits through. Additional exemplary objects that may be found in operating room 100 are provided with reference to FIG. 2.

The cameras (e.g., cameras 102a, 102b, and 120) can be oriented toward one or more areas or objects of interest in the operating room. For example, one or more cameras can be oriented toward: the door such that they can capture images of the door, the operating table such that they can capture images of the operating table, the patient such that they can capture images of the patient, medical equipment (e.g., X-Ray device, anesthesia machine, staple gun, retractor, clamp, endoscope, electrocautery tool, fluid management system, waste management system, suction units, etc.) such that they can capture images of the medical equipment, surgical staff (e.g., surgeon, anesthesiologist, surgical assistant, scrub nurse, circulating nurse, registered nurse) such that they can capture images of the surgical staff, etc. Multiple cameras can be placed in different locations in the operating room such that they can collectively capture a particular area or object of interest from different perspectives. Some cameras can track a moving object. The one or more cameras can include PTZ cameras. The cameras can include cameras that can provide a video stream over a network. The one or more cameras can include a camera integrated into a surgical light in the operating room.

An aspect of the present disclosure is to monitor various aspects of the surgical environment to detect surgical milestones and to adjust a surgical schedule based on the detected milestones, among other follow-up actions described herein. The cameras (e.g., cameras 102a, 102b, 120) placed inside the operating room can capture images to detect one or more surgical milestones using one or more trained machine-learning models and, based on the detected milestones, a surgical schedule specifying timing information for one or more surgeries to occur in the operating room can be adjusted. Further, based upon detection of one or more surgical milestones the system may determine whether preparation of pre-operation patients should be accelerated or delayed, determine the availability of one or more medical resources for a scheduled surgical procedure, determine whether to schedule an emergency surgery in the operating room, determine whether the operating room is available for a next surgery, determine whether supplies need to be restocked, and/or determine whether sterilization processes can be accelerated or delayed. The system may generate an output (e.g., an alert) when certain milestones are detected. The system may record information (e.g., timing information, patient status, etc.) associated with a detected milestone in a database, such as an electronic medical record, for further processing and analysis as described herein.

It will be appreciated that the cameras can capture images of one or more surgical environments, such as medical care areas, e.g. one or more operating rooms. An aspect of the present disclosure is to monitor various aspects of the surgical environments to detect surgical milestones and to adjust a surgical schedule based on the detected milestones, among other follow-up actions described herein. The cameras (e.g., cameras 102a, 102b, 120) placed inside the one or more operating rooms can capture images to detect, in each operating room, one or more surgical milestones using one or more trained machine-learning models and, based on the detected milestones, a surgical schedule specifying timing information for one or more surgeries to occur in the one or more operating rooms can be adjusted. Further, based upon detection of one or more surgical milestones the system may determine whether preparation of pre-operation patients should be accelerated or delayed, determine the availability of one or more medical resources for a scheduled surgical procedure, determine whether to schedule an emergency surgery in one or more of the operating rooms, determine whether one or more of the operating rooms is available for a next surgery, determine whether supplies need to be restocked, and/or determine whether sterilization processes can be accelerated or delayed. The system may generate an output (e.g., an alert) when certain milestones are detected. The system may record information (e.g., timing information, patient status, etc.) associated with a detected milestone in a database, such as an electronic medical record, for further processing and analysis as described herein.

FIG. 2 illustrates an exemplary process 200 for adjusting a surgical schedule specifying timing information for one or more surgeries to occur in an operating room. Process 200 is performed, for example, using one or more electronic devices implementing a software platform. Process 200 can be performed using a client-server system, and the blocks of process 200 are divided up in any manner between the server and a client device. Alternatively, the blocks of process 200 are divided up between the server and multiple client devices. Alternatively, process 200 is performed using only a client device or only multiple client devices. In process 200, some blocks are, optionally, combined, the order of some blocks is, optionally, changed, and some blocks are, optionally, omitted. Additional steps may be performed in combination with the process 200. Accordingly, the operations as illustrated (and described in greater detail below) are exemplary by nature and, as such, should not be viewed as limiting.

At block 202, an exemplary system (e.g., one or more electronic devices) receives one or more images of the operating room, or rooms, captured by one or more cameras (e.g., cameras 102a and/or 102b in FIG. 1). The one or more images may include video frames. Alternatively, or additionally, the one or more images may include still images. As discussed above, the one or more cameras can be placed inside the operating room. The one or more cameras can be oriented toward: the door such that they can capture images of the door, the operating table such that they can capture images of the operating table, medical equipment (e.g., diagnostic imaging device, anesthesia machine, staple gun, retractor, clamp, endoscope, electrocautery tool) such that they can capture images of the medical equipment, surgical staff (e.g., surgeon, anesthesiologist, surgical assistant, nurse) such that they can capture images of the surgical staff, etc. Multiple cameras can be placed in different locations in the operating room such as they can collectively capture a particular area or object of interest from different perspectives. The one or more cameras can include PTZ cameras. The one or more cameras can include a camera integrated into a surgical light in the operating room.

Multiple cameras may be placed at different angles oriented toward a first door (e.g., a door the patient enters through) and/or a second door (e.g., a door sterile equipment and staff enter through) in the operating room, multiple cameras may be oriented toward the operating table from different angles, and one or more cameras may be oriented toward the surgical lights and the surgeon. Different cameras, depending on the orientation of the camera, may be associated with different models configured to detect different objects such that images captured by a given camera are processed by associated model(s), as described in detail below.

The one or more images can include images captured by one or more surgical device (e.g., endoscopes). By utilizing images captured by cameras generally installed in the operating room in conjunction with information from surgical devices, the system may provide a more accurate and realistic identification of surgical milestones and estimation of time between surgical milestones (e.g., surgery started, surgery closing). For example, a cholecystectomy may last anywhere from one to three hours (or even longer), depending on the patient, the anatomy, and the surgeon's skills. Therefore, being able to correctly identify all the surgical milestones specific to this procedure (using intra-operative endoscopic images among other types of images) may result in a more accurate overall remaining time estimation.

At block 204, the system detects one or more surgical milestones of a plurality of predefined surgical milestones using one or more trained machine-learning models based on the received one or more images. A surgical milestone can indicate the stage of progression through a surgical procedure. The plurality of predefined milestones can include: whether an operating room is ready, whether operating room setup has started, whether a medical staff member (e.g., the surgeon, the scrub nurse, the technician) is donning surgical attire (e.g., masks, gloves, caps, gowns), whether operating room equipment is being set up, whether the patient is brought in to the operating room, whether the patient is ready for intubation or anesthesia, whether a timeout is occurring, whether the timeout has occurred, whether the patient is intubated or anesthetized, whether the patient has been prepped and draped for surgery, whether the patient is ready for surgery, whether a surgery site prep is complete, whether a surgery has started, whether the surgery is closing, whether a dressing is applied to the patient, whether the surgery is stopped, whether the patient is brought out of the operating room, whether the operating room is being cleaned, whether the operating room is clean, or any combination thereof. It should be understood that the foregoing list of milestones is merely exemplary. There may be fewer, additional, or different predefined milestones, for instance, depending on a type of surgical procedure.

the system can use the one or more trained machine learning models to detect one or more detected objects or events, which are in turn used to determine the one or more surgical milestones (e.g., surgical time points, surgical phases). The one or more trained machine learning models can include an object detection algorithm, an object tracking algorithm, a video action detection algorithm, an anomaly detection algorithm, or any combination thereof.

The system can first use an object detection algorithm to detect a particular type of object in an image, and then use an object tracking algorithm to track the movement and/or status of the detected object in subsequent images. Using one or more object detection algorithms, the system may detect one or more objects and assign an object ID to each detected object. The one or more object detection algorithms can comprise machine-learning models such as a 2D convolutional neural network (CNN) or 3D-CNN (e.g., MobileNetV2, ResNet, MobileNetV3, CustomCNN). After the objects are detected, the system may then use one or more object tracking algorithms to track the movement of the detected objects. The one or more object tracking algorithms can comprise any computer-vision algorithms for tracking objects and can comprise non-machine-learning algorithms. The object tracking algorithm(s) may involve execution of more lightweight code than the object detection algorithm(s), thus improving efficiency and reducing latency for surgical milestone determination. An object detection algorithm can include an instance segmentation algorithm, which can be configured to simultaneously perform classification (e.g., determining what type of object an image depicts), semantic segmentation (e.g., determining what pixels in the image belong to the object), and instance association (e.g., identifying individual instances of the same class; for example, person1 and person2). Additionally, in real-world scenes, a given visual object may be occluded by other objects. Although human vision systems can locate and recognize severely occluded objects with temporal context reasoning and prior knowledge, it may be challenging for classical video understanding systems to perceive objects in the heavily occluded video scenes. Accordingly, some examples of the present disclosure include machine-learning algorithms that take into account the temporal component of the video stream. For example, the system may perform spatial feature calibration and temporal fusion for effective one-stage video instance segmentation. As another example, the system may perform spatio-temporal contrastive learning for video instance segmentation. Additional information on these exemplary algorithms can be found, for example, in Li et al., "Spatial Feature Calibration and Temporal Fusion for Effective One-stage Video Instance Segmentation", arXiv:21 04. 05606v 1, available at https://doi.org/10.48550/arXiv.2104.05606, and Jiang et al., "STC: Spatio-Temporal Contrastive Learning for Video Instance Segmentation", arXiv:2202.03747v1, available at https://doi.org/10.48550/arXiv.2202.03747, both of which are incorporated herein by reference.

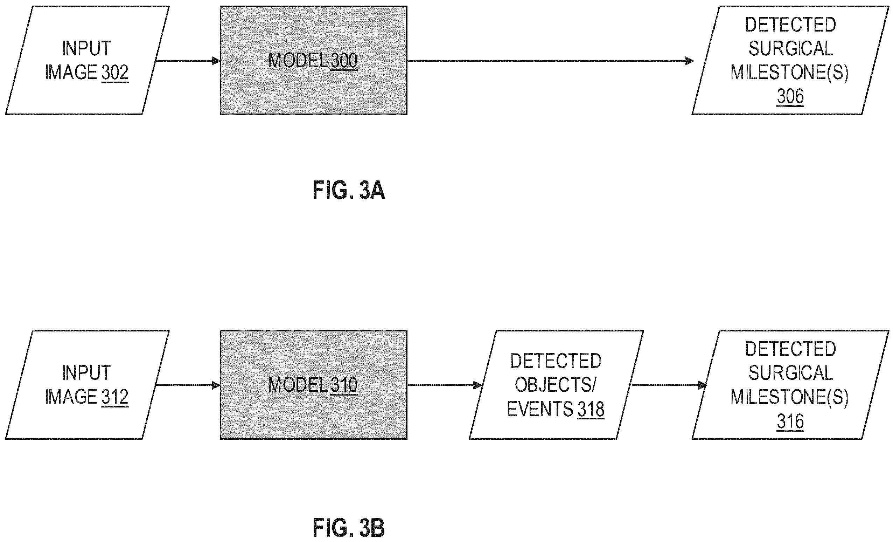

The tracked movement and/or status of one or more detected objects can then be used to determine events occurring in the operating room. For example, the system can first use an object detection model to detect a stretcher in an image and then use an object tracking algorithm to detect when the stretcher crosses door coordinates to determine that the stretcher is being moved into the operating room (i.e., an event). The one or more trained machine-learning models can be trained using a plurality of annotated images (e.g., annotated with labels of object(s) and/or event(s)). Further description of such machine learning models is provided below with reference to FIG. 3A.

An object that the system can detect can include physical items, persons, or parts thereof, located inside, entering, or leaving an operating room. In some examples, the object can include a stretcher, a patient, a surgeon, an anesthesiologist, the surgeon's hand, a surgical assistant, a scrub nurse, a technician, a nurse, a scalpel, sutures, a staple gun, a door to a sterile room, a door to a non-sterile corridor, a retractor, a clamp, an endoscope, an electrocautery tool, an intubation mask, a surgical mask, a C-Arm, an Endoscopic Equipment Stack, an anesthesia machine, an anesthesia cart, a fluid management system, a waste management system, a waste disposal receptacle, an operating table, surgical table accessories, an equipment boom, an anesthesia boom, an endoscopic equipment cart, surgical lights, a case cart, a sterile back table, a sterile mayo stand, a cleaning cart, an X-Ray device, an imaging device, a trocar, a surgical drape, operating room floor, EKG leads, ECG leads, bed linens, a blanket, a heating blanket, a lap belt, safety straps, a pulse oximeter, a blood pressure machine, an oxygen mask, an IV, or any combination thereof.

An event that the system can detect can include a status, change of status, and/or an action associated with an object. In some examples, the event can include whether the surgical lights are turned off, whether the operating table is vacant, whether the bed linens are wrinkled, whether the bed linens are stained, whether the operating table is wiped down, whether a new linen is applied to the operating table, whether a first sterile case cart is brought into the operating room, whether a new patient chart is created, whether instrument packs are distributed throughout the operating room, whether booms and suspended equipment are repositioned, whether the operating table is repositioned, whether a nurse physically exposes instrumentation by unfolding linen or paper, or opening instrumentation containers using a sterile technique, whether the scrub nurse entered the operating room, whether the technician entered the operating room, whether the scrub nurse is donning a gown, whether the circulating nurse is securing the scrub nurse's gown, whether the scrub nurse is donning gloves using the sterile technique, whether the sterile back table or the sterile mayo stand is being set with sterile instruments, whether the patient is wheeled into the operating room on a stretcher, whether the patient is wheeled into the operating room on a wheel chair, whether the patient walked into the operating room, whether the patient is carried into the operating room, whether the patient is transferred to the operating table, whether the patient is covered with the blanket, whether the lap belt is applied to the patient, whether the pulse oximeter is placed on the patient, whether the EKG leads are applied to the patient, whether the ECG leads are applied to the patient, whether the blood pressure cuff is applied to the patient, whether a surgical sponge and instrument count is conducted, whether a nurse announces a timeout, whether a surgeon announces a timeout, whether an anesthesiologist announces a timeout, whether activities are stopped for a timeout, whether the anesthesiologist gives the patient the oxygen mask, whether the patient is sitting and leaning over with the patient's back cleaned and draped, whether the anesthesiologist inspects the patient's anatomy with a long needle, whether the anesthesiologist injects medication into the patient's back, whether the anesthesiologist indicates that the patient is ready for surgery, whether the patient is positioned for a specific surgery, whether required surgical accessories are placed on a table, whether padding is applied to the patient, whether the heating blanket is applied to the patient, whether the safety straps are applied to the patient, whether a surgical site on the patient is exposed, whether the surgical lights are turned on, whether the surgical lights are positioned to illuminate the surgical site, whether the scrub nurse is gowning the surgeon, whether the scrub nurse is gloving the surgeon, whether skin antiseptic is applied, whether the surgical site is draped, whether sterile handles are applied to the surgical lights, whether a sterile team member is handing off tubing to a non-sterile team member, whether a sterile team member is handing off electrocautery to a non-sterile team member, whether the scalpel is handed to the surgeon, whether an incision is made, whether the sutures are handed to the surgeon, whether the staple gun is handed to the surgeon, whether the scrub nurse is handing a sponge to a sponge collection basin, whether an incision is closed, whether dressing is applied to cover a closed incision, whether the surgical lights are turned off, whether the anesthesiologist is waking the patient, whether the patient is returned to a supine position, whether extubation is occurring, whether instruments are being placed on the case cart, whether a garbage bag is being tied up, whether the bed linens are collected and tied up, whether the operating table surface is cleaned, whether the operating room floor is being mopped, whether the patient is being transferred to a stretcher, whether the patient is being brought out of the operating room, whether the surgical table is dressed with a clean linen, whether a second sterile case cart is brought into the operating room, or any combination thereof.

Instead of using trained machine-learning models to detect objects/events (which are then used to determine surgical milestones), the system can use trained machine-learning models to output surgical milestones directly. A trained machine-learning model of the one or more trained machine-learning models can be a machine-learning model (e.g., deep-learning model) trained using annotated surgical video information, where the annotated surgical video information includes annotations of at least one of the plurality of predefined surgical milestones. Further description of such machine learning models is provided below with reference to FIG. 3B.

The system may perform a spatial analysis (e.g., based on object detection/tracking as discussed above), a temporal analysis, or a combination thereof. The system may perform the temporal analysis using a temporal deep neural network (DNN), such as LSTM, Bi-LSTM, MSTCN, etc. The DNN may be trained using one or more training videos in which the start time and the end time of various surgical milestones are bookmarked. The temporal analysis may be used to predict remaining surgery duration, as discussed below.

The one or more trained machine-learning models used herein can comprise a trained neural network model, such as a 2D CNN, 3D-CNN, temporal DNN, etc. For example, the models may comprise ResNet50, AlexNet, Yolo, I3D ResNet 50, LSTM, MSTCN, etc. The one or more trained machine-learning models may comprise supervised learning models that are trained using annotated images such as human-annotated images. Additionally or alternatively, the one or more trained machine-learning model may comprise self-supervised learning models where a specially trained network can predict the remaining surgery duration, without relying on labeled images. As examples, a number of exemplary models are described in G. Yengera et al., "Less is More: Surgical Phase Recognition with Less Annotations through Self-Supervised Pre-training of CNN-LSTM Networks," arXiv: 1805.08569, available at https://arxiv.org/abs/1805.08569. For example, an exemplary model may utilize a self-supervised pre-training approach based on the prediction of remaining surgery duration (RSD) from laparoscopic videos. The RSD prediction task is used to pre-train a CNN and long short-term memory (LSTM) network in an end-to-end manner. The model may utilize all available data and reduces the reliance on annotated data, thereby facilitating the scaling up of surgical phase recognition algorithms to different kinds of surgeries. Another example model may comprise an end-to-end trained CNN-LSTM model for surgical phase recognition. It should be appreciated by one of ordinary skill in the art that other types of object detection algorithms, object tracking algorithms, video action detection algorithms, that provide sufficient performance and accuracy (e.g., in real time) can be used. The system can include machine-learning models associated with a family of architectures based on visual transformers, which may perform image recognition at scale. An exemplary framework is a Self-supervised Transformer with Energy-based Graph Optimization (STEGO) and may be capable of jointly discovering and segmenting objects without any human supervision. Building upon another self-supervised architecture, DINO, STEGO can distill pre-trained unsupervised visual features into semantic clusters using a novel contrastive loss. Additional information on visual transformers can be found, for example, in Caron et al., "An Image is Worth 16x16 Words: Transformers for Image Recognition at Scale", arXiv:2010.11929v2, available at https://doi.org/10.48550/arXiv.2010.11929, which is incorporated herein by reference. Additional information on DINO and STEGO can be found, for example, in Hamilton et al., "Unsupervised Semantic Segmentation by Distilling Feature Correspondences", arXiv:2203.08414vl, available at https://doi.org/10.48550/arXiv.2203.08414, and Caron et al., "Emerging Properties in Self-Supervised Vision Transformers", arXiv:2104.14294v2, available at https://doi.org/10.48550/arXiv.2104.14294, which are incorporated herein by reference.

Different cameras in the operating room, or rooms, depending on the placement and orientation, may be associated with different models configured to detect different objects, events, and/or milestones. In other words, images captured by a given camera may be processed by associated model(s). For example, a camera oriented towards an operating room door can be associated with a model configured to detect when the patient is brought in or out of the operating room and a model configured to detect door open and door closed states. The model may also return the object's (e.g., the door, the patient) position in each frame. As another example, the camera oriented towards the first door (i.e., the door the patient enters through) may be associated with a model configured to detect a stretcher, an open door, and a closed door, along with the coordinates of the detected objects. The camera oriented towards the second door (i.e., the door leading toward the sterile corridor) may be associated with a model configured to detect a sterile case cart entering the operating room and an unsterile case cart leaving through the #2 door at the end of the procedure. The camera oriented toward the patient may be associated with a model configured to detect the patient (a person), the attachment of equipment onto the patient and removal of medical equipment from the patient (e.g., Oxygen pulse monitor, blood pressure cuff, ECG leads, EKG leads, operating table, and intubation mask). The camera oriented towards the surgical lights may be associated with a model configured to detect when the lights are turned on or turned off, and the camera oriented toward the surgeon may be associated with a model configured to detect when an incision is made. It should be understood that the foregoing list is meant to be merely exemplary, and the one or more cameras may have any number of models associated for detecting objects, events, and/or surgical milestones.

At block 206, the system adjusts, based on the detected one or more surgical milestones, the surgical schedule specifying timing information for the one or more surgeries to occur in the operating room, or rooms. The system may further display the adjusted surgical schedule. Families of patients can be alerted to scheduling changes, for instance, by a communication system. In some examples, the communication system may include a Health Insurance Portability and Accountability Act (HIPAA) compliant dashboard in or outside the operating room. The communication system may generate SMS updates. Such alerts may serve to alleviate family concerns caused by unanticipated delays or other adjustments to the surgical schedule, in contrast to conventional systems that may be incapable of detecting surgical milestones, adjusting surgical schedules, and/or providing such alerts.

Adjusting the surgical schedule can include: obtaining an original schedule (e.g., obtained from one or more databases of an institutional system such as an EMR system, an EHR system, a HIS system), where the original schedule may be based on historical time information of the plurality of predefined surgical milestones; comparing the original schedule with time information of the one or more detected surgical milestones; and updating, based on the comparison, the surgical schedule. The historical timing information can include an average of the timing at which a surgical milestone is detected during a surgical procedure. For example, if the comparison of the original schedule indicates that a surgical milestone is taking longer than expected based on the historical average, the surgical schedule can be adjusted to reflect an anticipated delay in surgery completion. Similarly, if the comparison of the original schedule indicates that a surgical milestone has been completed more quickly than expected based on the historical average, the surgical schedule can be adjusted to reflect an anticipation that the surgery will be completed ahead of the original schedule. In some examples, the historical average is electronically stored/maintained. A historical average may be associated with a specific surgeon, a specific type of surgery, a specific phase of a specific type of surgery, a specific operating room, a specific hospital, a specific group of hospitals, or any combination thereof. In some examples, the specific surgeon or specific type of surgery may be identified using one or more machine-learning models such as the models described herein.

The system may additionally or alternatively use timing information associated with the people in the operating room when adjusting the surgical schedule. Specifically, the system can identify one or more people associated with a surgery in the operating room. The one or more people can include one or more surgeons performing the surgery, one or more people from the surgical crew, one or more medical staff, or any combination thereof. The system can identify the one or more people associated with the surgery using a machine-learning model. For example, a machine-learning model can be trained to receive an image and recognize one or more people (e.g., surgeons, nurses, anesthesiologists, technicians, assistants) that may be in the operating room during a surgery. During a surgery, one or more images may be captured (e.g., using cameras 102a, 102b, 120) and provided to the machine-learning model to identify who are performing the surgery in the operating room.

The system can be configured to identify the one or more people associated with the surgery by retrieving data associated with the surgery from a database. The database (e.g., an EMR system, an EHR system, a HIS system) can store various data associated with the surgery, such as the surgeon, the surgical crew, the patient, the procedure, etc.

The system can obtain timing information associated with the identified one or more people. For example, given a specific surgeon, the system can obtain timing information indicating how long the surgeon typically takes to complete a specific type of procedure, a specific surgical milestone, etc. Timing information may be obtained from a database (e.g., an EMR system, an EHR system, a HIS system). In some examples, the system can adjust the surgical schedule based on the timing information.

The system may adjust the surgical schedule based on a weighted combination of the timing information associated with the identified one or more people and the detected one or more surgical milestones. For example, if the system determines (e.g., based on the detected surgical milestones) that the surgery is on track to finish on time or approximately on track (e.g., the time deviation is within a predefined threshold), the system may assign a larger weight to the timing information associated with the identified one or more people to determine the adjusted schedule. On the other hand, if the system determines (e.g., based on the detected surgical milestones) that the surgery is not on track to finish (e.g., the time deviation exceeds the predefined threshold), the system may assign a smaller weight to the timing information such that the detected milestones are weighted more heavily to determine the amount of adjustment to the schedule. The system may adjust the surgical schedule for one operating room or for a plurality of operating rooms.

The system may determine, based upon the detected one or more surgical milestones, whether preparation of pre-operation patients should be accelerated or delayed. In accordance with a determination that preparation of pre-operation patients should be accelerated, preparation of pre-operation patients can be accelerated. In accordance with a determination that preparation of pre-operation patients should be delayed, preparation of pre-operation patients can be delayed. For instance, if it is determined that a surgery is proceeding ahead of schedule based upon the detected one or more surgical milestones, surgical preparation of a pre-operation patient awaiting surgery in the operating room can be accelerated. Similarly, it is determined that a surgery is proceeding behind schedule based upon the detected one or more surgical milestones, surgical preparation of a pre-operation patient awaiting surgery in the operating room can be delayed. The determination to accelerate or delay preparation of a pre-operation patient may be made based upon a comparison between the original schedule with time information of the one or more detected surgical milestones. The historical timing information can include averages of the timing at which a surgical milestone is detected during a surgical procedure. The historical timing information may be electronically stored/maintained (e.g., in a database), and the historical timing information may be associated with a specific surgeon, a specific type of surgery, a specific phase of a specific type of surgery, a specific operating room, a specific hospital, a specific group of hospitals, or any combination thereof. The specific surgeon or specific type of surgery may be identified using one or more machine-learning models such as the models described herein. The updated schedule for preparing pre-operation patients can be outputted by the communication system described herein. The system may determine, based upon the detected one or more surgical milestones, whether a surgery should be rescheduled to a different operating room.

The system can further determine the availability of medical resources for other surgeries. For instance, the system may identify, based upon the one or more images captured by the one or more cameras, one or more medical resources in use during a surgery. The system may then determine, based on the identified one or more medical resources in use during the surgery, the availability of the identified one or more medical resources for a scheduled surgical procedure. The system may monitor all operating rooms in a hospital to track available medical resources in the hospital. The system may monitor only a subset of operating rooms (e.g., the ones in use) and can determine the availability of medical resources based on image analysis as well as existing inventory information in one or more databases. The one or more medical resources may be medical equipment (e.g., an Endoscopic Equipment Stack, an anesthesia machine, an anesthesia cart, a fluid management system, a waste management system, etc.), surgical staff (e.g., a surgeon, surgical assistant, a scrub nurse, a technician, a nurse, an anesthesiologist, etc.), or any combination thereof. If the inventory of a medical resource is known by the system, the availability of that medical resource (e.g., a C-Arm) may be ascertained for use in scheduled procedures. For example, upon identification of a medical resource in use during a surgery, the system may determine that the resource is not available for other procedures (e.g., future, concurrent, and/or emergency medical procedures). As another example, the system may, based on an identified medical resource in use during a surgery and the known inventory of the medical resource, schedule supply restocking of the medical resource. The system may automatically generate and/or place an order to restock the medical resources.

The system may generate an output (e.g., an alert) based on the determined availability of the identified one or more medical resources. For example, the system may generate an alert that a member of the surgical staff (e.g., a surgeon) is in a surgery beyond the expected duration of the surgery and thus not available for other surgeries. As another example, the system may identify that an article of medical equipment is in use and generate an alert that the identified medical equipment is not available for any other procedures for the duration of its use.

The surgical schedule may be adjusted based on the determined availability of the identified one or more medical resources. For example, if the system identifies a medical resource and determines, based on the identification, that the medical resource is not available for a scheduled procedure requiring that medical resource. Accordingly, the system may delay the scheduled procedure until the identified resource becomes available.

The system may determine whether a detected surgical milestone of the detected one or more surgical milestones is a charting event. A charting event may be an event for which information associated with the detected milestone is recorded in a database (e.g., an electronic medical record, patient chart). In accordance with a determination that the detected surgical milestone is a charting event, the system can record the detected surgical milestone in a database. The database may be an electronic medical record (EMR). In some examples, charting events may include whether the patient is brought into the operating room, whether a timeout is occurring, whether the timeout has occurred, whether a surgery has started, whether the surgery is stopped, and whether the patient is brought out of the operating room.

The system may determine whether a detected surgical milestone of the detected one or more surgical milestones is an alerting event. An alerting event may be an event for which an alert may be generated containing information associated with the milestone. In accordance with a determination that the detected surgical milestone is an alerting event, the system can generate an alert. Alerting events may include whether operating room setup has started, whether the patient is brought in to the operating room, whether the patient is ready for intubation or anesthesia, whether the patient is intubated or anesthetized, whether the patient has been prepped and draped for surgery, whether the patient is ready for surgery, whether a surgery has started, whether the surgery is closing, whether a dressing is applied to the patient, whether the surgery is stopped, whether the patient is brought out of the operating room, and whether the operating room is clean. For example, in accordance with a determination that the patient is ready for intubation and/or anesthesia, the system can generate an alert that a timeout should be conducted. As another example, the system may recognize whether a timeout has been conducted and issue an alert if a surgery is started without a timeout being conducted. It should be understood that the aforementioned surgical milestones identified as charting events and/or alerting events are meant to be exemplary and should not be interpreted as limiting. In some examples, additional, fewer, or different milestones may be determined to be charting and/or alerting events.

The system can be configured to provide an output (e.g., displays a generated alert, displays a notification, displays a status indicator such as the current surgical milestone/phase, displays an original surgical schedule, displays an adjusted surgical schedule, displays information associated with a detected object, event, or milestone, etc.) on a display in the operating room. As shown in the example in FIG. 1, the output (e.g., an alert that a timeout protocol should be conducted, an alert that the timeout protocol has been conducted, an alert that surgery is closing, a display of an updated surgical schedule, or any other alerts described herein) can be displayed on the display 112 in the operating room 100. The system can display the output on a display in a monitoring area. As shown in the example in FIG. 1, an output (e.g., an alert that surgery has started, an alert that surgery is running ahead of or behind schedule, an adjusted schedule, etc.) in operating room 100 can be displayed on the display 126, which can be a monitor at a central control center for monitoring multiple operating rooms. The system can display the output as a message (e.g., a text message such as an SMS message, a notification) on a mobile device. As shown in the example in FIG. 1, an output can be displayed on the mobile phone 124. While the outputs shown in FIG. 1 are textual alerts, it should be appreciated that the output can additionally or alternatively comprise a graphical component, a visual component (e.g., a flashing light), an audio component, a haptic component, or any combination thereof. The output can be provided based on userconfigurable settings (e.g., at a user-specified frequency). The system may use different devices and/or formats for displaying different types of outputs. For example, a status indicator showing the current surgical milestone or phase may be displayed on the display 112 or 126, while an alert about an emergency surgery may be sent to the surgeon's mobile device as a SMS message. It should be understood that the aforementioned outputs are meant to be exemplary and not limiting. The system may be configured to provide a variety of different or additional outputs.

The system can automatically update, based on the one or more detected objects or events, a surgical timeout checklist specifying a list of surgical protocol action items. The surgical timeout is a chance for the entire team to stop and confirm that everything is in order prior to commencing surgery. A surgical timeout protocol includes a checklist that is confirmed usually by staff during the surgical timeout. The visual or audio recognition of events in the operating room can be used to partially or fully populate the surgical timeout checklist automatically using the techniques described herein, and the surgical staff's role may be to conduct and complete remaining timeout items and/or verify the validity of recognized timeout activities recorded by the system. As such, the system may decrease the time and/or complexity associated with conventional surgical timeout protocol.

The system can determine, based upon the detected one or more surgical milestones, whether to schedule an emergency surgery in the operating room, or one of the plurality of operating rooms. In accordance with a determination that an emergency surgery should be scheduled, the surgery can be scheduled. In accordance with a determination that an emergency surgery should not be scheduled, the system may forego scheduling the emergency surgery. For example, the system may detect whether a surgery has started in the operating room and thus determine that the room is not available for an emergency surgery. As such, an emergency surgery may not be scheduled. As another example, the system may detect that medical resources required for the emergency surgery are currently in use and thus determine that the emergency surgery should not be scheduled. A determination that an emergency surgery should not be scheduled can be used in making a decision to divert a patient to an alternative operating room or an alternative medical facility, for instance, if the patient is currently being transported to the hospital by emergency response personnel. The system may schedule the emergency surgery or forego scheduling the emergency surgery. The system may generate a recommendation to either schedule or forego scheduling the emergency surgery, and a human may schedule or forego scheduling the surgery. The system may generate a recommendation to cancel a scheduled elective surgical procedure to schedule the emergency surgery. For example, the system can access schedules in one or more databases (e.g., of the HIS system) and identify procedures from the schedule that are elective and relatively low-priority (e.g., an ACL repair procedure) that can be scheduled to a later time.

The system may determine, based on the detected one or more surgical milestones, whether the operating room is available for a next surgery. In accordance with a determination that an operating room is available for surgery, the system may generate an alert indicating that the room is available for the next surgery. In accordance with a determination that an operating room is not available for surgery, the system may forego generation of the alert indicating that the room is available for the next surgery. The system may determine, based on the detected one or more surgical milestones, that the surgical workflow has entered a workflow phase or completed a workflow phase. In accordance with a determination that a surgical workflow phase has been entered, the system may generate an alert indicating that the phase has been entered. In accordance with a determination that a surgical workflow phase has been completed, the system may generate an alert indicating that the phase has been completed.

Based on the detected surgical milestones in a surgery, the system can automatically generate a detailed timeline of the procedure, and record it in a database. These records can be used, in turn, for training a machine-learning algorithm for predicting future surgeries' durations based on patient data and surgery type, thus making scheduling process more efficient. For example, the machine-learning algorithm may be configured to receive information about a surgery (e.g., surgery type, patient data) and output a prediction of how long the surgery and/or each surgical milestone may take. The model may be trained using historical data stored in the database.

FIGS. 3A and 3B illustrate exemplary machine-learning models that can be used to detect surgical milestone(s), in accordance with some examples. Both models 300 and 310 can receive an input image (e.g., an image received in block 202). The model(s) 300 can be configured to directly output one or more surgical milestones depicted in the input image. In contrast, the model(s) 310 can be configured to output one or more detected objects or events 318, which in turn can be used by the system to determine one or more surgical milestones depicted in the input image. Models 300 and 310 are described in detail below.

With reference to FIG. 3A, a model 300 is configured to receive an input image 302 and directly output an output 306 indicative of one or more surgical milestones detected in the input image 302. The model 300 can be trained using a plurality of training images depicting the one or more surgical milestones. For example, the model 300 can be trained using a plurality of annotated training images. Each of the annotated images can depict a scene of an operating room and include one or more labels indicating surgical milestone(s) depicted in the scene. The plurality of annotated training images can comprises a video in which surgical milestones are bookmarked. At least some of the annotated images can be captured in the same operating room (e.g., operating room 100) for which the model will be deployed. During training, the model receives each image of the annotated images and provides an output indicative of detected surgical milestone(s). The output is compared against the labels associated with the image. Based on the comparison, the model 300 can be updated (e.g., via a backpropagation process).

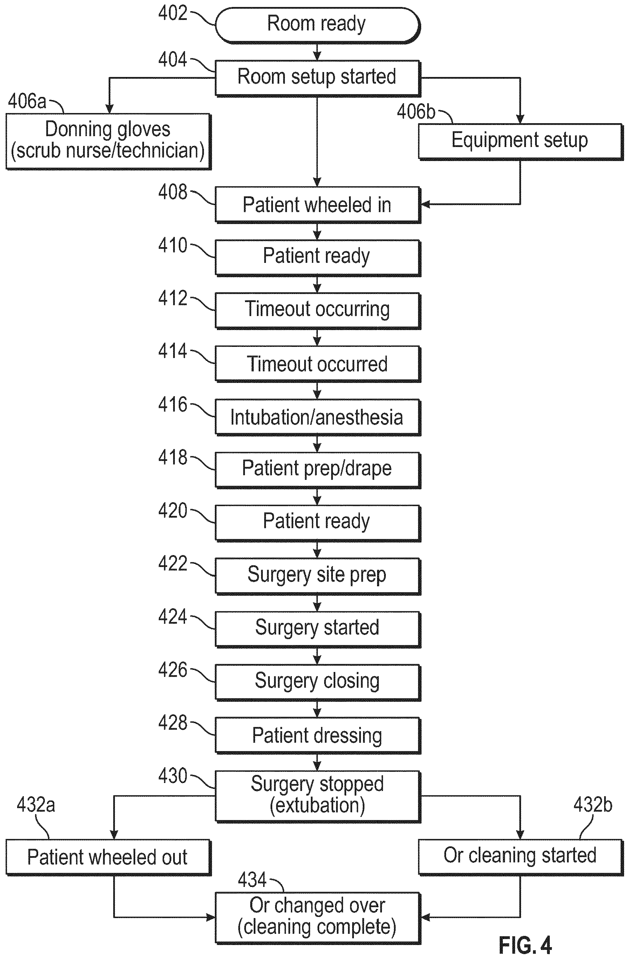

With reference to FIG. 3B, a model 310 is configured to receive an input image 312 and output one or more detected objects and/or events 318 depicted in the input image 312. Based on the one or more detected objects and/or events 318, the system can determine, as output 316, one or more surgical milestones detected in the input image 312, as described with reference to FIG. 4. The one or more machine learning models can be trained using a plurality of training images depicting the one or more objects and/or events. For example, the model 310 can be trained using a plurality of annotated training images. Each of the annotated images can depict a scene of an operating room and include one or more labels indicating objects and/or events depicted in the scene. In some examples, at least some of the annotated images are captured in the same operating room (e.g., operating room 100) for which the model will be deployed. During training, the model receives each image of the annotated images and provides an output indicative of one or more detected objects and/or events. The output is compared against the labels associated with the image. Based on the comparison, the model 300 can be updated (e.g., via a backpropagation process).

FIG. 4 illustrates an exemplary series of predefined surgical milestones 400 to be identified by the system during a surgery. It should be understood that some blocks in exemplary series 400 are, optionally, combined, the order of some blocks is, optionally, changed, and some blocks are, optionally, omitted. Additional surgical milestones may be included in combination with the process 400. For instance, many milestones throughout the surgery are surgery dependent according to specialty (e.g., orthopedics, plastic surgery, cardiac, neurological), and also depend on what surgery is being performed within each specialty. For example, a surgical milestone involving the use of a specific surgical equipment may be applicable to one type of surgery but not another type of surgery. Some blocks in exemplary series 400 are further described as alerting events and/or charting events. It should be understood that additional or different blocks or surgical milestones may be alerting and or charting events than those noted below. Accordingly, the operations as illustrated (and described in greater detail below) are exemplary by nature and, as such, should not be viewed as limiting.

The system may detect a first predefined surgical milestone at block 402 directed to whether the operating room is ready. A determination that the operating room is ready may be performed directly by one or more machine-learning models, or based on one or more objects/events detected by one or more machine-learning models, such as whether the operating room table is empty, whether the surgical lights are turned off, whether the bed linens are wrinkled, whether the operating table is wiped down, whether the operating room floor is mopped, whether a new linen is applied to the operating table, or any combination thereof. For instance, following the previous surgical procedure, the cleaning team would typically change over the room for the next surgery. To change over the room, the cleaning team may remove the used linen from the operating table, wipe down the operating table, and apply a new linen to the operating table, among other tasks. If the room has been changed over, the system may identify that the operating room is ready for the next surgical procedure.

Following a detection of the first surgical milestone at block 402, the system may proceed to the second surgical milestone at block 404, which is directed to whether the operating room setup has started. A determination of whether the operating room setup has started may be performed directly by one or more machine-learning models, or based on one or more objects/events detected by one or more machine-learning models, such as: whether a first sterile case cart is brought into the operating room, whether a new patient chart is created, or any combination thereof. For instance, before commencing a surgery, a nurse may bring in a new case cart with instruments wrapped in a sterile linen, sterile paper, or metal instrumentation case, and a nurse will begin a new patient chart (e.g., using an electronic device). The new patient chart can be be part of an electronic medical record, and the system may record charting events in the patient chart. If the system detects that these events have occurred and/or are occurring it may identify that operating room setup has started.

Following a detection of the second surgical milestone at block 404, the system may optionally proceed to the third surgical milestone at block 406a, which is directed to whether the scrub nurse or the technician are donning gloves. A determination of whether the scrub nurse or technician are donning gloves may be performed directly by one or more machine-learning models, or based on one or more objects/events detected by one or more machine-learning models, such as: whether the scrub nurse entered the operating room, whether the technician entered the operating room, whether the scrub nurse is donning a gown, whether the circulating nurse is securing the scrub nurse's gown, whether the scrub nurse is donning gloves using the sterile technique, whether the sterile back table or the sterile mayo stand is being set with sterile instruments, or any combination thereof.

Following a detection of the second surgical milestone at block 404, the system may optionally proceed to the fourth surgical milestone at block 406b, which is directed to whether operating room equipment is being set up. A determination of whether operating room equipment is being set up may be performed directly by one or more machine-learning models, or based on one or more objects/events detected by one or more machine-learning models, such as: whether instrument packs are distributed throughout the operating room, whether booms and suspended equipment are repositioned, whether surgical lights are repositioned, whether medical monitors are repositioned, whether the operating table is repositioned, whether instruments and equipment were opened using a sterile technique, whether a nurse physically exposes instrumentation by unfolding linen or paper, or any combination thereof. For example, after the new case cart is brought into the operating room, the scrub nurse and technician will garner their protective equipment (e.g., gloves), and begin placing the required medical resources (e.g., medical equipment including surgical tools, surgical supplies, the operating table, etc.) in their proper location throughout the operating room.

After block 404, 406a, and/or 406b, the system may proceed to the fifth surgical milestone at block 408, which is directed to whether the patient is being brought into the operating room. A determination of whether the patient is brought into the operating room may be performed directly by one or more machine-learning models, or based on one or more objects/events detected by one or more machine-learning models, such as: whether the patient is wheeled into the operating room on a stretcher, whether the patient is wheeled into the operating room on a wheel chair, whether the patient walked into the operating room, or whether the patient is carried into the operating room. The patient being brought into the operating room may be determined to be an alerting event, and the surgeon and other surgical staff may be alerted that the patient was brought into the operating room. Depending on the procedure, and on the condition of the patient and/or the convention for a geographic region or country, the method of bringing the patient into the operating room may vary. For instance, in some examples, a child may be carried into the operating room while an adult may be wheeled in on a stretcher. The system may be able to distinguish between the method of entry and generate an output specifying how the patient was brought into the operating room. Whether the patient is being brought into the operating room may also be a charting event. The system may record information related to the patient being brought into the operating room (e.g., timing, method of entry, etc.), in a database, for instance in an electronic medical record.

Following a detection of the fifth surgical milestone at block 408, the system may proceed to the sixth surgical milestone at block 410, which is directed to whether the patient is ready for intubation and anesthesia. A determination of whether the patient is ready for intubation and anesthesia may be performed directly by one or more machine-learning models, or based on one or more objects/events detected by one or more machine-learning models, such as: whether the patient is covered with the blanket, whether the lap belt is applied to the patient, whether the pulse oximeter is placed on the patient, whether the EKG leads are applied to the patient, whether the ECG leads are applied to the patient, whether the blood pressure cuff is applied to the patient, or any combination thereof. Some of these activities may have occurred in pre-op. The order in which the aforementioned devices (e.g., EKG leads, ECG leads, blood pressure cuff, pulse oximeter, etc.) may vary based on the type of surgery. For instance, the order may vary depending on whether local, regional, or general anesthetic is being applied. As another example, some anesthesiologists may begin administration of drugs (e.g., a sedative) prior to arrival in the OR but full anesthesia occurs in the OR. The circulating nurse may apply the aforementioned devices to the patient. A determination of whether the patient is ready for intubation and anesthesia may be an alerting event. For example, an alert may be generated reminding the surgical team to conduct a timeout prior intubating and anesthetizing the patient.

Following a detection of the sixth surgical milestone at block 410, the system may proceed to the seventh surgical milestone at block 412, which is directed to whether a timeout is occurring. A determination of whether a timeout has occurred may be performed directly by one or more machine-learning models, or based on one or more objects/events detected by one or more machine-learning models, such as: whether the nurse, anesthesiologist, or surgeon announces timeout, whether the surgical staff stops activities to participate in the timeout protocol, whether the surgical staff makes eye contact with the conductor, or any combination thereof. The determination that a timeout was conducted may be a charting event and information related to the timeout (e.g. timing information) may be recorded in a database, for instance, an electronic medical record.

Following a detection of the seventh surgical milestone at block 412, the system may proceed to the eighth surgical milestone at block 414, which is directed to whether a timeout has occurred. A determination of whether a timeout has occurred may be performed directly by one or more machine-learning models, or based on one or more objects/events detected by one or more machine-learning models, such as: whether the nurse, anesthesiologist, or surgeon announced a timeout, whether the surgical staff stopped activities to participate in the timeout protocol, whether the surgical staff made eye contact with the conductor, or any combination thereof. The determination that a timeout was conducted may be a charting event and information related to the timeout (e.g. timing information) may be recorded in a database, for instance, an electronic medical record.

Following a detection of the eighth surgical milestone at block 414, the system may proceed to the ninth surgical milestone at block 416, which is directed to whether the patient is intubated or anesthetized. A determination that the patient is intubated or anesthetized may be performed directly by one or more machine-learning models, or based on one or more objects/events detected by one or more machine-learning models, such as: whether the anesthesiologist has given the patient the oxygen mask (e.g., to pre-oxygenate the patient prior to intubation), whether the anesthesiologist is using a laryngoscope to insert an endotracheal tube into the patient, whether the patient is sitting and leaning over with the patient's back cleaned and draped, whether the anesthesiologist inspects the patient's anatomy with a long needle, whether the anesthesiologist injects medication into the patient's back, whether the anesthesiologist indicates that the patient is ready for surgery, or any combination thereof. A determination that the patient is intubated or anesthetized may be determined to be an alerting event, and the surgeon may be alerted that the patient is ready for prepping and draping. For instance, following completion of the timeout protocol, the procedure may proceed to intubation and anesthesia. The anesthesiologist and/or nurse anesthetist/anesthesia assistant will hold an O2 mask over the patient's face, commencing the induction anesthesia phase of the procedure. Alternatively, for regional anesthesia, the anesthesiologist and/or nurse anesthetist/anesthesia assistant may sit the patient up with legs over the edge of the operating table/bed for a Spinal/Epidural. As such, if the system detects one or more the aforementioned events occurring it may determine that the patient is being/has been intubated and/or anesthetized and may generate the aforementioned alert.

Following a detection of the ninth surgical milestone at block 416, the system may proceed to the tenth surgical milestone at block 418, which is directed to whether the patient has been prepped and draped for surgery. A determination that the patient has been prepped and draped for surgery may be performed directly by one or more machine-learning models, or based on one or more objects/events detected by one or more machine-learning models, such as: patient positioning (e.g., whether a patient is positioned for a specific surgery), whether required surgical accessories are placed on a table, whether surgical table accessories that were not already installed on a table are added to the table, whether padding is applied to the patient, whether a heating blanket is applied to the patient, whether safety straps are applied to the patient, whether a surgical site on the patient is exposed, or any combination thereof. The prepping and draping process may vary according to the type of surgery. For instance, different portions of the patient's body will be prepped and draped according to the planned incision locations. In some examples, a determination that the patient is prepped and draped for surgery may be an alerting event, and the surgical team may be alerted that the patient is prepped and draped for surgery. In some examples, upon receiving the alert that the patient is prepped and draped for surgery the surgeons may begin scrubbing.

Following a detection of the tenth surgical milestone at block 418, the system may proceed to the eleventh surgical milestone at block 420, which is directed to whether the patient is ready for surgery. A determination of whether the patient is ready for surgery may be performed directly by one or more machine-learning models, or based on one or more objects/events detected by one or more machine-learning models, such as: whether the surgical lights are turned on, whether the surgical lights are positioned to illuminate the surgical site, whether the scrub nurse is gowning the surgeon, whether the scrub nurse is gloving the surgeon, or any combination thereof. A determination that the patient is ready for surgery may be an alerting event, and the surgical team may be alerted that the patient is ready for surgery. A determination that the patient is ready for surgery may be preceded by a determination that surgical tools (i.e., all electrocautery, suction, etc.) are being handed off the surgical field and that surgery is ready to begin. A determination that surgical tools (i.e., all electrocautery, suction, etc.) are being handed off the surgical field and that surgery is ready to begin may be based on whether sterile handles are applied to surgical lights, whether the sterile team hands off tubing (suction, irrigation, etc.) to non-sterile team members for connection to equipment, whether the sterile team hands off electrocautery to non-sterile team members for connection to equipment, or any combination thereof.

Following a detection of the eleventh surgical milestone at block 420, the system may proceed to the twelfth surgical milestone at block 422, which is directed to whether a surgery site prep is complete. A determination that surgery site prep is complete may be performed directly by one or more machine-learning models, or based on one or more objects/events detected by one or more machine-learning models, such as: whether skin antiseptic is applied, whether the medical staff has removed/shaven hair from the patient, whether the surgical site is draped, or any combination thereof.

Following a detection of the twelfth surgical milestone at block 422, the system may proceed to the thirteenth surgical milestone at block 424, which is directed to whether a surgery has started. A determination that surgery has started may be performed directly by one or more machine-learning models, or based on one or more objects/events detected by one or more machine-learning models, such as: whether the scalpel is handed to the surgeon, whether a sponge and/or sponges are handed to the surgeon, whether an incision(s) is made, whether trocars have been inserted into the patient, or any combination thereof. For instance, in some examples, a scalpel and two sponges may be handed to the surgeon, an incision or poke incisions (holes) may be made by the surgeon. The system may detect one or more of these events and determine that the surgery has started. A determination that the surgery has started may be a charting event and recorded in a database, for instance an electronic medical record. In some examples, the surgery start time will be recorded in the database. A determination that the surgery has begun may also be determined to be an alerting event, and an alert may be generated indicating that surgery has begun (e.g., to operating room coordinators, family members, etc.).

Following a detection of the thirteenth surgical milestone at block 424, the system may proceed to the fourteenth surgical milestone at block 426, which is directed to whether the surgery is closing. A determination that surgery is closing may be performed directly by one or more machine-learning models, or based on one or more objects/events detected by one or more machine-learning models, such as: whether the sutures are handed to the surgeon, whether the staple gun is handed to the surgeon, whether the scrub nurse is handing a sponge to a sponge collection basin, whether an incision is closed, whether surgical lights are turned off or any combination thereof. The surgeon may be handed sutures and/or other surgical tools to close the incision while a sponge count and/or instrument count is occurring. The system may detect one or more of these events and determine that the surgery is closing. Two staff members (one sterile, one non-sterile) may count sponges and other surgical tools in a collection basin. Counting may be prioritized by item category, for instance, sponges may be counted first, then sharps, then metals. A determination that the surgery is closing may be an alerting event, and alert may be generated indicating that surgery is closing.

Following a detection of the fourteenth surgical milestone at block 426, the system may proceed to the fifteenth surgical milestone at block 428, which is directed to whether a dressing is applied to the patient. A determination that a dressing is applied to the patient may be performed directly by one or more machine-learning models, or based on one or more objects/events detected by one or more machine-learning models, such as: whether dressing is applied to cover a closed incision, whether the incision is wrapped, whether sterile drapes are removed, or any combination thereof. A determination that a dressing is applied to the patient may be an alerting event. For instance, an alert may be generated indicating that a dressing has been applied to inform cleaning staff, operating room coordinators, family, etc., indicating the stage of the surgery.

Following a detection of the fifteenth surgical milestone at block 428, the system may proceed to the sixteenth surgical milestone at block 430, which is directed to whether the surgery has stopped. A determination that surgery has stopped may be performed directly by one or more machine-learning models, or based on one or more objects/events detected by one or more machine-learning models, such as: whether the anesthesiologist is waking the patient, whether the patient is returned to a supine position (e.g., legs removed from stirrups, etc.), whether extubation is occurring, or any combination thereof. A determination that the surgery has stopped may be a charting event and recorded in a database, for instance an electronic medical record. The surgery stop time may be recorded in the database. A determination that the surgery has stopped may also be determined to be an alerting event, and an alert may be generated indicating that surgery has stopped (e.g., to operating room coordinators, family members, etc.).

Following a detection of the sixteenth surgical milestone at block 430, the system may optionally proceed to the seventeenth surgical milestone at block 432a, which is directed to whether the patient is brought out of the operating room. A determination that a patient is brought out of the operating room may be performed directly by one or more machine-learning models, or based on one or more objects/events detected by one or more machine-learning models, such as: whether the patient is being transferred to a stretcher, whether the patient is transferred to a bed, whether the patient is being brought out of the operating room. A determination that the patient is brought out of the operating room may be an alerting event. For instance, an alert may be generated informing the family that the patient is exiting the operating room and being transferred to a recovery area. A determination that the patient is brought out of the operating room may be a charting event that is recorded in a database, for instance an electronic medical record. As an example, the time that the patient leaves the operating room and the door through which the patient exits may be recorded in the electronic medical record.

Following a detection of the sixth surgical milestone at block 430, the system may optionally proceed to the eighteenth surgical milestone at block 432b, which is directed to whether the operating room is being cleaned. A determination that the operating room is being cleaned may be performed directly by one or more machine-learning models, or based on one or more objects/events detected by one or more machine-learning models, such as: whether instruments (e.g., non-sterile, soiled, and/or used instruments) are being placed on the case cart, whether a garbage bag is being tied up, whether the bed linens are collected and tied up, whether the operating table surface is cleaned, whether the operating room floor is being mopped, whether the cleaning staff is wiping items off, or any combination thereof. After the patient is brought out of the operating room, cleaning staff may enter the operating room and the operating room is cleaned (i.e., changed over). To change over the operating room, the cleaning staff may, in addition to those items noted above with reference to the first surgical milestone, remove dirty Anesthesia equipment, surgical instruments, carts, and garbage through a door to the "soil room," the location of which may vary according to the medical facility. A determination that the operating room is being cleaned may be an alerting event. For instance, an alert may be generated informing the next surgical team, or operating room coordinators, that cleaning has begun and the room will soon be available for the next surgical procedure.

Following 432a and/or 432b, the system may proceed to the nineteenth surgical milestone at block 434, which is directed to whether the operating room is clean. A determination that the operating room is clean may be performed directly by one or more machine-learning models, or based on one or more objects/events detected by one or more machine-learning models, such as: whether the surgical table is dressed with a clean linen, whether a second sterile case cart is brought into the operating room, or any combination thereof. A determination that the operating room is clean may be an alerting event. For instance, an alert may be generated indicating that the next case cart should be brought into the operating room. The system may detect that the next case cart is brought into the operating room through an operating room door, and the system may begin identifying a predefined series of milestones for the next surgical procedure.

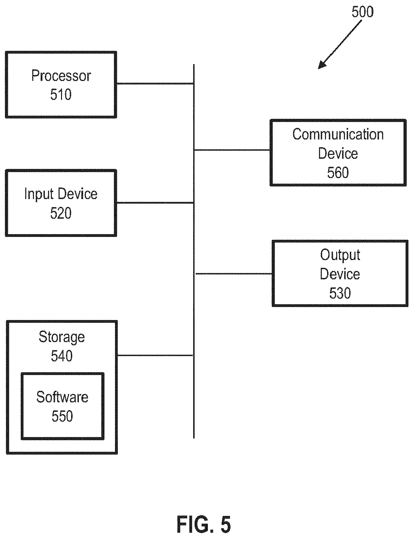

The operations described herein are optionally implemented by components depicted in FIG. 5. FIG. 5 illustrates an example of a computing device in accordance with one example. Device 500 can be a host computer connected to a network. Device 500 can be a client computer or a server. As shown in FIG. 5, device 500 can be any suitable type of microprocessor-based device, such as a personal computer, workstation, server or handheld computing device (portable electronic device) such as a phone or tablet. The device can include, for example, one or more of processor 510, input device 520, output device 530, storage 540, and communication device 560. Input device 520 and output device 530 can generally correspond to those described above, and can either be connectable or integrated with the computer.

Input device 520 can be any suitable device that provides input, such as a touch screen, keyboard or keypad, mouse, or voice-recognition device. Output device 530 can be any suitable device that provides output, such as a touch screen, haptics device, or speaker.

Storage 540 can be any suitable device that provides storage, such as an electrical, magnetic or optical memory including a RAM, cache, hard drive, or removable storage disk. Communication device 560 can include any suitable device capable of transmitting and receiving signals over a network, such as a network interface chip or device. The components of the computer can be connected in any suitable manner, such as via a physical bus or wirelessly.

Software 550, which can be stored in storage 540 and executed by processor 510, can include, for example, the programming that embodies the functionality of the present disclosure (e.g., as embodied in the devices as described above).

Software 550 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 540, that can contain or store programming for use by or in connection with an instruction execution system, apparatus, or device.

Software 550 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic or infrared wired or wireless propagation medium.

Device 500 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T5 lines, cable networks, DSL, or telephone lines.

Device 500 can implement any operating system suitable for operating on the network. Software 550 can be written in any suitable programming language, such as C, C++, Java or Python. In various examples, application software embodying the functionality of the present disclosure can be deployed in different configurations, such as in a client/server arrangement or through a Web browser as a Web-based application or Web service, for example.

The disclosure will now be further described by the following numbered embodiments which are to be read in connection with the preceding paragraphs, and which do not limit the disclosure. The features, options and preferences as described above apply also to the following embodiments.

Embodiment 1. A method for adjusting a surgical schedule specifying timing information for one or more surgeries to occur in an operating room, the method comprising:
receiving one or more images of the operating room captured by one or more cameras;
detecting one or more surgical milestones of a plurality of predefined surgical milestones using one or more trained machine-learning models based on the received one or more images, wherein the one or more machine learning models are trained using a plurality of training images depicting the one or more surgical milestones; and
adjusting, based on the detected one or more surgical milestones, the surgical schedule specifying timing information for the one or more surgeries to occur in the operating room.

Embodiment 2. The method of Embodiment 1, further comprising: displaying the adjusted surgical schedule.

Embodiment 3. The method of any of Embodiments 1-2, wherein detecting the one or more surgical milestones comprises:
obtaining, from the one or more trained machine-learning models, one or more detected objects or events; and
determining, based upon the one or more detected objects or events, the one or more surgical milestones.

Embodiment 4. The method of any of Embodiments 1-3, wherein adjusting the surgical schedule includes:
obtaining an original schedule, wherein the original schedule is based on historical time information of the plurality of predefined surgical milestones;
comparing the original schedule with time information of the one or more detected surgical milestones; and
updating, based on the comparison, the surgical schedule.

Embodiment 5. The method of any of Embodiments 1-4, further comprising:
determining, based upon the detected one or more surgical milestones, whether preparation of pre-operation patients should be accelerated or delayed; and
in accordance with a determination that preparation of pre-operation patients should be accelerated, accelerating preparation of pre-operation patients;
in accordance with a determination that preparation of pre-operation patients should be delayed, delaying preparation of pre-operation patients.

Embodiment 6. The method of any of Embodiments 1-5, further comprising:
identifying, based upon the one or more images, one or more medical resources in use during a surgery; and
determining, based on the identified one or more medical resources in use during the surgery, the availability of the identified one or more medical resources for a scheduled surgical procedure.

Embodiment 7. The method of Embodiment 6, wherein the one or more medical resources comprise medical equipment, surgical staff, or any combination thereof.

Embodiment 8. The method of Embodiment 6 or 7, further comprising: generating an alert based on the determined availability of the identified one or more medical resources.

Embodiment 9. The method of Embodiment 6, 7 or 8, wherein adjusting the surgical schedule comprises adjusting the surgical schedule based on the determined availability of the identified one or more medical resources.

Embodiment 10. The method of any of Embodiments 1-9, further comprising:
determining whether a detected surgical milestone of the detected one or more surgical milestones is a charting event or an alerting event;
in accordance with a determination that the detected surgical milestone is a charting event, recording the detected surgical milestone in a database; and
in accordance with a determination that the detected surgical milestone is an alerting event, generating an alert.

Embodiment 11. The method of Embodiment 10, wherein the database is an electronic medical record.

Embodiment 12. The method of Embodiment 3, or any of Embodiments 4-11 as far as dependent from Embodiment 3, further comprising: automatically updating, based on the one or more detected objects or events, a surgical timeout checklist specifying a list of surgical action items.

Embodiment 13. The method of any of Embodiments 1-12, further comprising: determining, based upon the detected one or more surgical milestones, whether to schedule an emergency surgery in the operating room;
in accordance with a determination that an emergency surgery should be scheduled, scheduling the surgery;
in accordance with a determination that an emergency surgery should not be scheduled, foregoing scheduling the emergency surgery.

Embodiment 14. The method of any of Embodiments 1-13, further comprising:
determining, based on the detected one or more surgical milestones, whether the operating room is available for a next surgery;
in accordance with a determination that an operating room is available for surgery, generating an alert indicating that the room is available for the next surgery;
in accordance with a determination that an operating room is not available for surgery, foregoing generation of the alert indicating that the room is available for the next surgery.

Embodiment 15. The method of Embodiment 3, or any of Embodiments 4-14 as far as dependent from Embodiment 3, wherein the one or more objects include:
a stretcher,
a patient,
a surgeon,
an anesthesiologist,
the surgeon's hand,
a surgical assistant,
a scrub nurse,
a technician,
a nurse,
a scalpel,
sutures,
a staple gun,
a door to a sterile room,
a door to a non-sterile corridor,
a retractor,
a clamp,
an endoscope,
an electrocautery tool,
an intubation mask,
a surgical mask,
a C-Arm,
an Endoscopic Equipment Stack,
an anesthesia machine,
an anesthesia cart,
a fluid management system,
a waste management system,
a waste disposal receptacle,
an operating table,
surgical table accessories,
an equipment boom,
an anesthesia boom,
an endoscopic equipment cart,
surgical lights,
a case cart,
a sterile back table,
a sterile mayo stand,
a cleaning cart,
an X-Ray device,
an imaging device,
a trocar,
a surgical drape,
operating room floor,
EKG leads,
ECG leads,
bed linens,
a blanket,
a heating blanket,
a lap belt,
safety straps,
a pulse oximeter,
a blood pressure machine,
an oxygen mask,
an IV,
or any combination thereof.

Embodiment 16. The method of Embodiment 3, or any of Embodiments 4-15 as far as dependent from Embodiment 3, wherein the one or more events include:
whether the surgical lights are turned off,
whether the operating table is vacant,
whether the bed linens are wrinkled,
whether the bed linens are stained,
whether the operating table is wiped down,
whether a new linen is applied to the operating table,
whether a first sterile case cart is brought into the operating room,
whether a new patient chart is created,
whether instrument packs are distributed throughout the operating room,
whether booms and suspended equipment are repositioned,
whether the operating table is repositioned,
whether a nurse physically exposes instrumentation by unfolding linen or paper, or opening instrumentation containers using a sterile technique,
whether the scrub nurse entered the operating room,
whether the technician entered the operating room,
whether the scrub nurse is donning a gown,
whether the circulating nurse is securing the scrub nurse's gown,
whether the scrub nurse is donning gloves using the sterile technique,
whether the sterile back table or the sterile mayo stand is being set with sterile instruments,
whether the patient is wheeled into the operating room on a stretcher,
whether the patient is wheeled into the operating room on a wheel chair,
whether the patient walked into the operating room,
whether the patient is carried into the operating room,
whether the patient is transferred to the operating table,
whether the patient is covered with the blanket,
whether the lap belt is applied to the patient,
whether the pulse oximeter is placed on the patient,
whether the EKG leads are applied to the patient,
whether the ECG leads are applied to the patient,
whether the blood pressure cuff is applied to the patient,
whether a surgical sponge and instrument count is conducted,
whether a nurse announces a timeout,
whether a surgeon announces a timeout,
whether an anesthesiologist announces a timeout,
whether activities are stopped for a timeout,
whether the anesthesiologist gives the patient the oxygen mask,
whether the patient is sitting and leaning over with the patient's back cleaned and draped,
whether the anesthesiologist inspects the patient's anatomy with a long needle,
whether the anesthesiologist injects medication into the patient's back,
whether the anesthesiologist indicates that the patient is ready for surgery,
whether the patient is positioned for a specific surgery,
whether required surgical accessories are placed on a table,
whether padding is applied to the patient,
whether the heating blanket is applied to the patient,
whether the safety straps are applied to the patient,
whether a surgical site on the patient is exposed,
whether the surgical lights are turned on,
whether the surgical lights are positioned to illuminate the surgical site,
whether the scrub nurse is gowning the surgeon,
whether the scrub nurse is gloving the surgeon,
whether skin antiseptic is applied,
whether the surgical site is draped,
whether sterile handles are applied to the surgical lights,
whether a sterile team member is handing off tubing to a non-sterile team member,
whether a sterile team member is handing off electrocautery to a non-sterile team member,
whether the scalpel is handed to the surgeon,
whether a sponge is handed to the surgeon,
whether an incision is made,
whether the sutures are handed to the surgeon,
whether the staple gun is handed to the surgeon,
whether the scrub nurse is handing a sponge to a sponge collection basin,
whether an incision is closed,
whether dressing is applied to cover a closed incision,
whether the surgical lights are turned off,
whether the anesthesiologist is waking the patient,
whether the patient is returned to a supine position,
whether extubation is occurring,
whether instruments are being placed on the case cart,
whether a garbage bag is being tied up,
whether the bed linens are collected and tied up,
whether the operating table surface is cleaned,
whether the operating room floor is being mopped,
whether the patient is being transferred to a stretcher,
whether the patient is being brought out of the operating room,
whether the surgical table is dressed with a clean linen,
whether a second sterile case cart is brought into the operating room,
or any combination thereof.

Embodiment 17. The method of any of Embodiments 1-16, wherein the plurality of predefined milestones include:
whether an operating room is ready,
whether operating room setup has started,
whether the scrub nurse or the technician are donning gloves,
whether operating room equipment is being set up,
whether the patient is brought into the operating room,
whether the patient is ready for intubation or anesthesia,
whether a timeout is occurring,
whether the timeout has occurred,
whether the patient is intubated or anesthetized,
whether the patient has been prepped and draped for surgery,
whether the patient is ready for surgery,
whether a surgery site prep is complete,
whether a surgery has started,
whether the surgery is closing,
whether a dressing is applied to the patient,
whether the surgery is stopped,
whether the patient is brought out of the operating room,
whether the operating room is being cleaned,
whether the operating room is clean,
or any combination thereof.

Embodiment 18. The method of Embodiment 17, wherein determining whether the operating room is ready is based on:
whether the operating room table is empty,
whether the surgical lights are turned off,
whether the bed linens are wrinkled,
whether the operating table is wiped down,
whether the operating room floor is mopped, or
whether a new linen is applied to the operating table.

Embodiment 19. The method of Embodiment 17 or 18, wherein determining whether the patient is prepped and draped for surgery is based on:
whether the patient is positioned for a specific surgery,
whether required surgical accessories are placed on a table,
whether padding is applied to the patient,
whether the heating blanket is applied to the patient,
whether the safety straps are applied to the patient, or
whether a surgical site on the patient is exposed.

Embodiment 20. The method of any of Embodiments 1-19, wherein a trained machine-learning model of the one or more trained machine-learning models is an object detection algorithm, a video action detection algorithm, a surgical milestone detection algorithm, an anomaly detection algorithm, or any combination thereof.

Embodiment 21. The method of any of Embodiments 1-19, wherein a trained machine-learning model of the one or more trained machine-learning models is a neural network model.

Embodiment 22. The method of any of Embodiments 1-21, wherein the one or more trained machine-learning models are trained using a plurality of annotated images.

Embodiment 23. The method of Embodiment 22, wherein a trained machine-learning model of the one or more trained machine-learning models is a deep learning model trained using annotated surgical video information, wherein the annotated surgical video information includes annotations of at least one of the plurality of predefined surgical milestones.

Embodiment 24. The method of any of Embodiments 1-23, further comprising: training, based on the detected one or more surgical milestones, a machine-learning model configured to predict timing information of a future surgery.

Embodiment 25. The method of Embodiment 24, wherein the timing information comprises a predicted duration of the future surgery, a predicted duration of a surgical milestone of the future surgery, or any combination thereof.

Embodiment 26. The method of any of Embodiments 1-25, further comprising:
identifying one or more people associated with a surgery in the operating room; and
obtaining timing information associated with the identified one or more people.

Embodiment 27. The method of Embodiment 26, where the surgical schedule is adjusted based on a weighted combination of the timing information associated with the identified one or more people and the detected one or more surgical milestones.

Embodiment 28. The method of Embodiment 26 or 27, wherein identifying the one or more people comprises: retrieving data associated with the surgery from a database.

Embodiment 29. The method of Embodiment 26, 27 or 28, wherein identifying the one or more people comprises: detecting the one or more people using a trained machine-learning model based on the received one or more images.

Embodiment 30. The method of any of Embodiments 1-29, wherein the plurality of predefined surgical milestones comprises one or more preoperative milestones, one or more intraoperative milestones, and one or more postoperative milestones.

Embodiment 31. A system for adjusting a surgical schedule specifying timing information for one or more surgeries to occur in an operating room, comprising:
one or more processors;
a memory; and
one or more programs, wherein the one or more programs are stored in the memory and configured to be executed by the one or more processors, the one or more programs including instructions for:
   receiving one or more images of the operating room captured by one or more cameras;
   detecting one or more surgical milestones of a plurality of predefined surgical milestones using one or more trained machine-learning models based on the received one or more images, wherein the one or more machine learning models are trained using a plurality of training images depicting the one or more surgical milestones; and
   adjusting, based on the detected one or more surgical milestones, the surgical schedule specifying timing information for the one or more surgeries to occur in the operating room.

Embodiment 32. The system of Embodiment 31, wherein the one or more programs further include instructions for: displaying the adjusted surgical schedule.

Embodiment 33. The system of any of Embodiments 31-32, wherein detecting the one or more surgical milestones comprises:
obtaining, from the one or more trained machine-learning models, one or more detected objects or events; and
determining, based upon the one or more detected objects or events, the one or more surgical milestones.

Embodiment 34. The system of any of Embodiments 31-33, wherein adjusting the surgical schedule includes:
obtaining an original schedule, wherein the original schedule is based on historical time information of the plurality of predefined surgical milestones;
comparing the original schedule with time information of the one or more detected surgical milestones; and
updating, based on the comparison, the surgical schedule.

Embodiment 35. The system of any of Embodiments 31-34, wherein the one or more programs further include instructions for:
determining, based upon the detected one or more surgical milestones, whether preparation of pre-operation patients should be accelerated or delayed; and
in accordance with a determination that preparation of pre-operation patients should be accelerated, accelerating preparation of pre-operation patients;
in accordance with a determination that preparation of pre-operation patients should be delayed, delaying preparation of pre-operation patients.

Embodiment 36. The system of any of Embodiments 31-35, wherein the one or more programs further include instructions for:
identifying, based upon the one or more images, one or more medical resources in use during a surgery; and
determining, based on the identified one or more medical resources in use during the surgery, the availability of the identified one or more medical resources for a scheduled surgical procedure.

Embodiment 37. The system of Embodiment 36, wherein the one or more medical resources comprise medical equipment, surgical staff, or any combination thereof.

Embodiment 38. The system of Embodiment 36 or 37, wherein the one or more programs further include instructions for: generating an alert based on the determined availability of the identified one or more medical resources.

Embodiment 39. The system of Embodiment 36, 37 or 38, wherein adjusting the surgical schedule comprises adjusting the surgical schedule based on the determined availability of the identified one or more medical resources.

Embodiment 40. The system of any of Embodiments 31-39, wherein the one or more programs further include instructions for:
determining whether a detected surgical milestone of the detected one or more surgical milestones is a charting event or an alerting event;
in accordance with a determination that the detected surgical milestone is a charting event, recording the detected surgical milestone in a database; and
in accordance with a determination that the detected surgical milestone is an alerting event, generating an alert.

Embodiment 41. The system of Embodiment 40, wherein the database is an electronic medical record.

Embodiment 42. The system of Embodiment 33, or any of Embodiments 34-41 as far as dependent from Embodiment 33, wherein the one or more programs further include instructions for: automatically updating, based on the one or more detected objects or events, a surgical timeout checklist specifying a list of surgical action items.

Embodiment 43. The system of any of Embodiments 31-42, wherein the one or more programs further include instructions for: determining, based upon the detected one or more surgical milestones, whether to schedule an emergency surgery in the operating room;
in accordance with a determination that an emergency surgery should be scheduled, scheduling the surgery;
in accordance with a determination that an emergency surgery should not be scheduled, foregoing scheduling the emergency surgery.

Embodiment 44. The system of any of Embodiments 31-43, wherein the one or more programs further include instructions for:
determining, based on the detected one or more surgical milestones, whether the operating room is available for a next surgery;
in accordance with a determination that an operating room is available for surgery, generating an alert indicating that the room is available for the next surgery;
in accordance with a determination that an operating room is not available for surgery, foregoing generation of the alert indicating that the room is available for the next surgery.

Embodiment 45. The system of Embodiment 33, or any of Embodiments 34-44 as far as dependent from Embodiment 33, wherein the one or more objects include:
a stretcher,
a patient,
a surgeon,
an anesthesiologist,
the surgeon's hand,
a surgical assistant,
a scrub nurse,
a technician,
a nurse,
a scalpel,
sutures,
a staple gun,
a door to a sterile room,
a door to a non-sterile corridor,
a retractor,
a clamp,
an endoscope,
an electrocautery tool,
an intubation mask,
a surgical mask,
a C-Arm,
an Endoscopic Equipment Stack,
an anesthesia machine,
an anesthesia cart,
a fluid management system,
a waste management system,
a waste disposal receptacle,
an operating table,
surgical table accessories,
an equipment boom,
an anesthesia boom,
an endoscopic equipment cart,
surgical lights,
a case cart,
a sterile back table,
a sterile mayo stand,
a cleaning cart,
an X-Ray device,
an imaging device,
a trocar,
a surgical drape,
operating room floor,
EKG leads,
ECG leads,
bed linens,
a blanket,
a heating blanket,
a lap belt,
safety straps,
a pulse oximeter,
a blood pressure machine,
an oxygen mask,
an IV,
or any combination thereof.

Embodiment 46. The system of Embodiment 33, or any of Embodiments 34-45 as far as dependent from Embodiment 33, wherein the one or more events include:
whether the surgical lights are turned off,
whether the operating table is vacant,
whether the bed linens are wrinkled,
whether the bed linens are stained,
whether the operating table is wiped down,
whether a new linen is applied to the operating table,
whether a first sterile case cart is brought into the operating room,
whether a new patient chart is created,
whether instrument packs are distributed throughout the operating room,
whether booms and suspended equipment are repositioned,
whether the operating table is repositioned,
whether a nurse physically exposes instrumentation by unfolding linen or paper, or opening instrumentation containers using a sterile technique,
whether the scrub nurse entered the operating room,
whether the technician entered the operating room,
whether the scrub nurse is donning a gown,
whether the circulating nurse is securing the scrub nurse's gown,
whether the scrub nurse is donning gloves using the sterile technique,
whether the sterile back table or the sterile mayo stand is being set with sterile instruments,
whether the patient is wheeled into the operating room on a stretcher,
whether the patient is wheeled into the operating room on a wheel chair,
whether the patient walked into the operating room,
whether the patient is carried into the operating room,
whether the patient is transferred to the operating table,
whether the patient is covered with the blanket,
whether the lap belt is applied to the patient,
whether the pulse oximeter is placed on the patient,
whether the EKG leads are applied to the patient,
whether the ECG leads are applied to the patient,
whether the blood pressure cuff is applied to the patient,
whether a surgical sponge and instrument count is conducted,
whether a nurse announces a timeout,
whether a surgeon announces a timeout,
whether an anesthesiologist announces a timeout,
whether activities are stopped for a timeout,
whether the anesthesiologist gives the patient the oxygen mask,
whether the patient is sitting and leaning over with the patient's back cleaned and draped,
whether the anesthesiologist inspects the patient's anatomy with a long needle,
whether the anesthesiologist injects medication into the patient's back,
whether the anesthesiologist indicates that the patient is ready for surgery,
whether the patient is positioned for a specific surgery,
whether required surgical accessories are placed on a table,
whether padding is applied to the patient,
whether the heating blanket is applied to the patient,
whether the safety straps are applied to the patient,
whether a surgical site on the patient is exposed,
whether the surgical lights are turned on,
whether the surgical lights are positioned to illuminate the surgical site,
whether the scrub nurse is gowning the surgeon,
whether the scrub nurse is gloving the surgeon,
whether skin antiseptic is applied,
whether the surgical site is draped,
whether sterile handles are applied to the surgical lights,
whether a sterile team member is handing off tubing to a non-sterile team member,
whether a sterile team member is handing off electrocautery to a non-sterile team member,
whether the scalpel is handed to the surgeon,
whether a sponge is handed to the surgeon,
whether an incision is made,
whether the sutures are handed to the surgeon,
whether the staple gun is handed to the surgeon,
whether the scrub nurse is handing a sponge to a sponge collection basin,
whether an incision is closed,
whether dressing is applied to cover a closed incision,
whether the surgical lights are turned off,
whether the anesthesiologist is waking the patient,
whether the patient is returned to a supine position,
whether extubation is occurring,
whether instruments are being placed on the case cart,
whether a garbage bag is being tied up,
whether the bed linens are collected and tied up,
whether the operating table surface is cleaned,
whether the operating room floor is being mopped,
whether the patient is being transferred to a stretcher,
whether the patient is being brought out of the operating room,
whether the surgical table is dressed with a clean linen,
whether a second sterile case cart is brought into the operating room,
or any combination thereof.

Embodiment 47. The system of any of Embodiments 31-46, wherein the plurality of predefined milestones include:
whether an operating room is ready,
whether operating room setup has started,
whether the scrub nurse or the technician are donning gloves,
whether operating room equipment is being set up,
whether the patient is brought into the operating room,
whether the patient is ready for intubation or anesthesia,
whether a timeout is occurring,
whether the timeout has occurred,
whether the patient is intubated or anesthetized,
whether the patient has been prepped and draped for surgery,
whether the patient is ready for surgery,
whether a surgery site prep is complete,
whether a surgery has started,
whether the surgery is closing,
whether a dressing is applied to the patient,
whether the surgery is stopped,
whether the patient is brought out of the operating room,
whether the operating room is being cleaned,
whether the operating room is clean,
or any combination thereof.

Embodiment 48. The system of Embodiment 47, wherein determining whether the operating room is ready is based on:
whether the operating room table is empty,
whether the surgical lights are turned off,
whether the bed linens are wrinkled,
whether the operating table is wiped down,
whether the operating room floor is mopped, or
whether a new linen is applied to the operating table.

Embodiment 49. The system of Embodiment 47 or 48, wherein determining whether the patient is prepped and draped for surgery is based on:
whether the patient is positioned for a specific surgery,
whether required surgical accessories are placed on a table,
whether padding is applied to the patient,
whether the heating blanket is applied to the patient,
whether the safety straps are applied to the patient, or
whether a surgical site on the patient is exposed.

Embodiment 50. The system of any of Embodiments 31-49, wherein a trained machine-learning model of the one or more trained machine-learning models is an object detection algorithm, a video action detection algorithm, a surgical milestone detection algorithm, an anomaly detection algorithm, or any combination thereof.

Embodiment 51. The system of any of Embodiments 31-49, wherein a trained machine-learning model of the one or more trained machine-learning models is a neural network model.

Embodiment 52. The system of any of Embodiments 31-51, wherein the one or more trained machine-learning models are trained using a plurality of annotated images.

Embodiment 53. The system of Embodiment 52, wherein a trained machine-learning model of the one or more trained machine-learning models is a deep learning model trained using annotated surgical video information, wherein the annotated surgical video information includes annotations of at least one of the plurality of predefined surgical milestones.

Embodiment 54. The system of any of Embodiments 31-53, wherein the one or more programs further include instructions for: training, based on the detected one or more surgical milestones, a machine-learning model configured to predict timing information of a future surgery.

Embodiment 55. The system of Embodiment 54, wherein the timing information comprises a predicted duration of the future surgery, a predicted duration of a surgical milestone of the future surgery, or any combination thereof.

Embodiment 56. The system of any of Embodiments 31-55, wherein the one or more programs further include instructions for:
identifying one or more people associated with a surgery in the operating room; and
obtaining timing information associated with the identified one or more people.

Embodiment 57. The system of Embodiment 56, where the surgical schedule is adjusted based on a weighted combination of the timing information associated with the identified one or more people and the detected one or more surgical milestones.

Embodiment 58. The system of Embodiment 56 or 57, wherein identifying the one or more people comprises: retrieving data associated with the surgery from a database.

Embodiment 59. The system of Embodiment 56, 57 or 58, wherein identifying the one or more people comprises: detecting the one or more people using a trained machine-learning model based on the received one or more images.

Embodiment 60. The system of any of Embodiments 31-59, wherein the plurality of predefined surgical milestones comprises one or more preoperative milestones, one or more intraoperative milestones, and one or more postoperative milestones.

Embodiment 61. A non-transitory computer-readable storage medium storing one or more programs, the one or more programs comprising instructions, which when executed by one or more processors of an electronic device, cause the electronic device to perform any of the methods of Embodiments 1-30.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims.

The foregoing description, for purpose of explanation, has been described with reference to specific examples. However, the illustrative discussions above are not intended to be exhaustive or to limit the invention to the precise forms disclosed. Many modifications and variations are possible in view of the above teachings. The examples were chosen and described in order to best explain the principles of the techniques and their practical applications. Others skilled in the art are thereby enabled to best utilize the techniques and various examples with various modifications as are suited to the particular use contemplated.

## Claims

1. A method for adjusting a surgical schedule specifying timing information for one or more surgeries to occur in an operating room, the method comprising:
receiving one or more images of the operating room captured by one or more cameras;
detecting one or more surgical milestones of a plurality of predefined surgical milestones using one or more trained machine-learning models based on the received one or more images, wherein the one or more machine learning models are trained using a plurality of training images depicting the one or more surgical milestones; and
adjusting, based on the detected one or more surgical milestones, the surgical schedule specifying timing information for the one or more surgeries to occur in the operating room; and optionally displaying the adjusted surgical schedule.

2. The method of claim 1, wherein detecting the one or more surgical milestones comprises:
obtaining, from the one or more trained machine-learning models, one or more detected objects or events; and
determining, based upon the one or more detected objects or events, the one or more surgical milestones.

3. The method of any of claims 1-2, wherein adjusting the surgical schedule includes:
obtaining an original schedule, wherein the original schedule is based on historical time information of the plurality of predefined surgical milestones;
comparing the original schedule with time information of the one or more detected surgical milestones; and
updating, based on the comparison, the surgical schedule.

4. The method of any of claims 1-3, further comprising:
identifying, based upon the one or more images, one or more medical resources, such as medical equipment and/or surgical staff, in use during a surgery; and
determining, based on the identified one or more medical resources in use during the surgery, the availability of the identified one or more medical resources for a scheduled surgical procedure, wherein adjusting the surgical schedule comprises adjusting the surgical schedule based on the determined availability of the identified one or more medical resources.

5. The method of claim 2, or any of claims 3-4 as far as dependent from claim 2, further comprising: automatically updating, based on the one or more detected objects or events, a surgical timeout checklist specifying a list of surgical action items.

6. The method of any of claims 1-5, further comprising: determining, based upon the detected one or more surgical milestones:
a) whether to schedule an emergency surgery in the operating room; and
in accordance with a determination that an emergency surgery should be scheduled, scheduling the surgery;
in accordance with a determination that an emergency surgery should not be scheduled, foregoing scheduling the emergency surgery; and/or
b) whether the operating room is available for a next surgery; and
in accordance with a determination that an operating room is available for surgery, generating an alert indicating that the room is available for the next surgery;
in accordance with a determination that an operating room is not available for surgery, foregoing generation of the alert indicating that the room is available for the next surgery.

7. The method of claim 2, or any of claims 3-6 as far as dependent from claim 2, wherein
the one or more objects include:
a stretcher,
a patient,
a surgeon,
an anesthesiologist,
the surgeon's hand,
a surgical assistant,
a scrub nurse,
a technician,
a nurse,
a scalpel,
sutures,
a staple gun,
a door to a sterile room,
a door to a non-sterile corridor,
a retractor,
a clamp,
an endoscope,
an electrocautery tool,
an intubation mask,
a surgical mask,
a C-Arm,
an Endoscopic Equipment Stack,
an anesthesia machine,
an anesthesia cart,
a fluid management system,
a waste management system,
a waste disposal receptacle,
an operating table,
surgical table accessories,
an equipment boom,
an anesthesia boom,
an endoscopic equipment cart,
surgical lights,
a case cart,
a sterile back table,
a sterile mayo stand,
a cleaning cart,
an X-Ray device,
an imaging device,
a trocar,
a surgical drape,
operating room floor,
EKG leads,
ECG leads,
bed linens,
a blanket,
a heating blanket,
a lap belt,
safety straps,
a pulse oximeter,
a blood pressure machine,
an oxygen mask,
an IV,
or any combination thereof;
and/or wherein the one or more events include:
whether the surgical lights are turned off,
whether the operating table is vacant,
whether the bed linens are wrinkled,
whether the bed linens are stained,
whether the operating table is wiped down,
whether a new linen is applied to the operating table,
whether a first sterile case cart is brought into the operating room,
whether a new patient chart is created,
whether instrument packs are distributed throughout the operating room,
whether booms and suspended equipment are repositioned,
whether the operating table is repositioned,
whether a nurse physically exposes instrumentation by unfolding linen or paper, or
opening instrumentation containers using a sterile technique,
whether the scrub nurse entered the operating room,
whether the technician entered the operating room,
whether the scrub nurse is donning a gown,
whether the circulating nurse is securing the scrub nurse's gown,
whether the scrub nurse is donning gloves using the sterile technique,
whether the sterile back table or the sterile mayo stand is being set with sterile instruments,
whether the patient is wheeled into the operating room on a stretcher,
whether the patient is wheeled into the operating room on a wheel chair,
whether the patient walked into the operating room,
whether the patient is carried into the operating room,
whether the patient is transferred to the operating table,
whether the patient is covered with the blanket,
whether the lap belt is applied to the patient,
whether the pulse oximeter is placed on the patient,
whether the EKG leads are applied to the patient,
whether the ECG leads are applied to the patient,
whether the blood pressure cuff is applied to the patient,
whether a surgical sponge and instrument count is conducted,
whether a nurse announces a timeout,
whether a surgeon announces a timeout,
whether an anesthesiologist announces a timeout,
whether activities are stopped for a timeout,
whether the anesthesiologist gives the patient the oxygen mask,
whether the patient is sitting and leaning over with the patient's back cleaned and draped,
whether the anesthesiologist inspects the patient's anatomy with a long needle,
whether the anesthesiologist injects medication into the patient's back,
whether the anesthesiologist indicates that the patient is ready for surgery,
whether the patient is positioned for a specific surgery,
whether required surgical accessories are placed on a table,
whether padding is applied to the patient,
whether the heating blanket is applied to the patient,
whether the safety straps are applied to the patient,
whether a surgical site on the patient is exposed,
whether the surgical lights are turned on,
whether the surgical lights are positioned to illuminate the surgical site,
whether the scrub nurse is gowning the surgeon,
whether the scrub nurse is gloving the surgeon,
whether skin antiseptic is applied,
whether the surgical site is draped,
whether sterile handles are applied to the surgical lights,
whether a sterile team member is handing off tubing to a non-sterile team member,
whether a sterile team member is handing off electrocautery to a non-sterile team member,
whether the scalpel is handed to the surgeon,
whether a sponge is handed to the surgeon,
whether an incision is made,
whether the sutures are handed to the surgeon,
whether the staple gun is handed to the surgeon,
whether the scrub nurse is handing a sponge to a sponge collection basin,
whether an incision is closed,
whether dressing is applied to cover a closed incision,
whether the surgical lights are turned off,
whether the anesthesiologist is waking the patient,
whether the patient is returned to a supine position,
whether extubation is occurring,
whether instruments are being placed on the case cart,
whether a garbage bag is being tied up,
whether the bed linens are collected and tied up,
whether the operating table surface is cleaned,
whether the operating room floor is being mopped,
whether the patient is being transferred to a stretcher,
whether the patient is being brought out of the operating room,
whether the surgical table is dressed with a clean linen,
whether a second sterile case cart is brought into the operating room,
or any combination thereof;
and/or wherein the plurality of predefined milestones include:
whether an operating room is ready,
whether operating room setup has started,
whether the scrub nurse or the technician are donning gloves,
whether operating room equipment is being set up,
whether the patient is brought in to the operating room,
whether the patient is ready for intubation or anesthesia,
whether a timeout is occurring,
whether the timeout has occurred,
whether the patient is intubated or anesthetized,
whether the patient has been prepped and draped for surgery,
whether the patient is ready for surgery,
whether a surgery site prep is complete,
whether a surgery has started,
whether the surgery is closing,
whether a dressing is applied to the patient,
whether the surgery is stopped,
whether the patient is brought out of the operating room,
whether the operating room is being cleaned,
whether the operating room is clean,
or any combination thereof;
optionally wherein determining whether the operating room is ready is based on:
whether the operating room table is empty,
whether the surgical lights are turned off,
whether the bed linens are wrinkled,
whether the operating table is wiped down,
whether the operating room floor is mopped, or
whether a new linen is applied to the operating table; and/or optionally wherein
determining whether the patient is prepped and draped for surgery is based on:
whether the patient is positioned for a specific surgery,
whether required surgical accessories are placed on a table,
whether padding is applied to the patient,
whether the heating blanket is applied to the patient,
whether the safety straps are applied to the patient, or
whether a surgical site on the patient is exposed.

8. The method of any of claims 1-7, wherein
a) a trained machine-learning model of the one or more trained machine-learning models is an object detection algorithm, a video action detection algorithm, a surgical milestone detection algorithm, an anomaly detection algorithm, or any combination thereof, or a neural network model; and/or
b) the one or more trained machine-learning models are trained using a plurality of annotated images,
optionally wherein a trained machine-learning model of the one or more trained machine-learning models is a deep learning model trained using annotated surgical video information, wherein the annotated surgical video information includes annotations of at least one of the plurality of predefined surgical milestones; and/or
c) the method further comprising: training, based on the detected one or more surgical milestones, a machine-learning model configured to predict timing information of a future surgery,
optionally wherein the timing information comprises a predicted duration of the future surgery, a predicted duration of a surgical milestone of the future surgery, or any combination thereof.

9. The method of any of claims 1-8, further comprising:
identifying one or more people associated with a surgery in the operating room; and
obtaining timing information associated with the identified one or more people;
wherein optionally
a) the surgical schedule is adjusted based on a weighted combination of the timing information associated with the identified one or more people and the detected one or more surgical milestones; and/or
b) identifying the one or more people comprises: retrieving data associated with the surgery from a database; and/or
c) identifying the one or more people comprises: detecting the one or more people using a trained machine-learning model based on the received one or more images.

10. A system for adjusting a surgical schedule specifying timing information for one or more surgeries to occur in an operating room, comprising:
one or more processors;
a memory; and
one or more programs, wherein the one or more programs are stored in the memory and configured to be executed by the one or more processors, the one or more programs including instructions for:
receiving one or more images of the operating room captured by one or more cameras;
detecting one or more surgical milestones of a plurality of predefined surgical milestones using one or more trained machine-learning models based on the received one or more images, wherein the one or more machine learning models are trained using a plurality of training images depicting the one or more surgical milestones; and
adjusting, based on the detected one or more surgical milestones, the surgical schedule specifying timing information for the one or more surgeries to occur in the operating room, wherein optionally the one or more programs further include instructions for: displaying the adjusted surgical schedule.

11. The system of claim 10, wherein
a) detecting the one or more surgical milestones comprises:
obtaining, from the one or more trained machine-learning models, one or more detected objects or events; and
determining, based upon the one or more detected objects or events, the one or more surgical milestones; and/or
b) adjusting the surgical schedule includes:
obtaining an original schedule, wherein the original schedule is based on historical time information of the plurality of predefined surgical milestones;
comparing the original schedule with time information of the one or more detected surgical milestones; and
updating, based on the comparison, the surgical schedule; and/or
c) the one or more programs further include instructions for:
determining, based upon the detected one or more surgical milestones, whether preparation of pre-operation patients should be accelerated or delayed; and
in accordance with a determination that preparation of pre-operation patients should be accelerated, accelerating preparation of pre-operation patients;
in accordance with a determination that preparation of pre-operation patients should be delayed, delaying preparation of pre-operation patients.

12. The system of any of claims 10-11, wherein the one or more programs further include instructions for:
identifying, based upon the one or more images, one or more medical resources, such as medical equipment and/or surgical staff, in use during a surgery; and
determining, based on the identified one or more medical resources in use during the surgery, the availability of the identified one or more medical resources for a scheduled surgical procedure;
wherein optionally adjusting the surgical schedule comprises adjusting the surgical schedule based on the determined availability of the identified one or more medical resources.

13. The system of any of claims 10-12, wherein
a) a trained machine-learning model of the one or more trained machine-learning models is an object detection algorithm, a video action detection algorithm, a surgical milestone detection algorithm, an anomaly detection algorithm, or any combination thereof, or a neural network model; and/or
b) wherein the one or more trained machine-learning models are trained using a plurality of annotated images,
optionally wherein a trained machine-learning model of the one or more trained machine-learning models is a deep learning model trained using annotated surgical video information, wherein the annotated surgical video information includes annotations of at least one of the plurality of predefined surgical milestones; and/or
c) wherein the one or more programs further include instructions for: training, based on the detected one or more surgical milestones, a machine-learning model configured to predict timing information of a future surgery.

14. The system of any of claims 10-13, wherein the one or more programs further include instructions for:
identifying one or more people associated with a surgery in the operating room; and
obtaining timing information associated with the identified one or more people;
wherein optionally
a) the surgical schedule is adjusted based on a weighted combination of the timing information associated with the identified one or more people and the detected one or more surgical milestones; and/or
b) identifying the one or more people comprises: retrieving data associated with the surgery from a database; and/or
c) identifying the one or more people comprises: detecting the one or more people using a trained machine-learning model based on the received one or more images.

15. A computer program product comprising one or more programs, the one or more programs comprising instructions, which when executed by one or more processors of an electronic device, cause the electronic device to perform any of the methods of claims 1-9.
